(19) Europäisches Patentamt
European Patent Office
Office européen des brevets

(11) EP 1 776 340 B1

(12) **FASCICULE DE BREVET EUROPEEN**

(45) Date de publication et mention de la délivrance du brevet:
**03.12.2008 Bulletin 2008/49**

(21) Numéro de dépôt: **05793766.6**

(22) Date de dépôt: **02.08.2005**

(51) Int Cl.:
***C07D 209/42*** *(2006.01)* ***A61K 31/404*** *(2006.01)*
***A61P 29/00*** *(2006.01)*

(86) Numéro de dépôt international:
**PCT/FR2005/002014**

(87) Numéro de publication internationale:
**WO 2006/024776 (09.03.2006 Gazette 2006/10)**

(54) **DERIVES DE N-(1 H-INDOLYL)-I H-IND0LE-2-CARB0XAMIDES, LEUR PREPARATION ET LEUR APPLICATION EN THERAPEUTIQUE**

N-(1 H-INDOLYL)-1 H- INDOL-2-CARBOXAMID-DERIVATE, DEREN HERSTELLUNG UND THERAPEUTISCHE VERWENDUNG DAMIT

N-(1H-INDOLYL)-H-INDOLE-2-CARB0XAMIDE DERIVATIVES, THEIR PREPARATION AND THEIR THERAPEUTIC USE

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **05.08.2004 FR 0408652**

(43) Date de publication de la demande:
**25.04.2007 Bulletin 2007/17**

(73) Titulaire: **Sanofi-Aventis**
**75013 Paris (FR)**

(72) Inventeurs:
- **DUBOIS, Laurent**
  **F-92350 Le Plessis-Robinson (FR)**
- **EVANNO, Yannick**
  **75013 Paris (FR)**
- **EVEN, Luc**
  **F-75015 Paris (FR)**

(74) Mandataire: **Gaslonde, Aude**
**sanofi-aventis**
**Département Brevets**
**174, Avenue de France**
**75013 Paris (FR)**

(56) Documents cités:
**WO-A-03/049702**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 776 340 B1

Printed by Jouve, 75001 PARIS (FR)

## Description

Dérivés de *N*-(1*H*-indolyl)-1*H*-indole-2-carboxamides, leur préparation et leur application en thérapeutique.

L'invention a pour objet des composés dérivés de *N*-(1*H*-indolyl)-1*H*-indole-2-carboxamides, qui présentent une activité antagoniste *in vitro* et *in vivo* pour les récepteurs de type TRPV1 (ou VR1).

On connaît déjà des composés décrits dans le document WO-A-03049702 utiles dans le traitement de maladies dans lesquelles les récepteurs de type VR1 sont impliqués.

Il existe toujours une nécessité de trouver et de développer des produits présentant une bonne activité in vivo.

L'invention répond à ce but en proposant des composés nouveaux, qui présentent une activité antagoniste in vitro et in vivo pour les récepteurs de type VR1.

Un premier objet de l'invention concerne les composés répondant à la formule générale (I) ci-après.

Un autre objet de l'invention concerne des procédés de préparation des composés de formule générale (I).

Un autre objet de l'invention concerne l'utilisation des composés de formule générale (I) notamment dans des médicaments ou dans des compositions pharmaceutiques.

Les composés de l'invention répondent à la formule générale (I) :

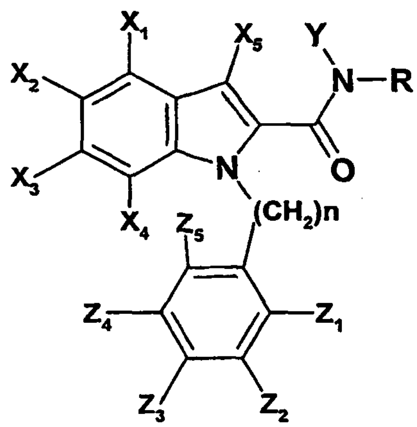

(I)

dans laquelle

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_1$-$C_6$-fluoroalkyle, $C_1$-$C_6$-alcoxy, $C_1$-$C_6$-fluoroalcoxy, cyano, C(O)$NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle ;

$X_5$ représente un atome d'hydrogène ou d'halogène ou un groupe $C_1$-$C_6$-alkyle, $C_1$-$C_6$-fluoroalkyle ;

R représente un groupe 4-, 5-, 6- ou 7-indolyle,

R = 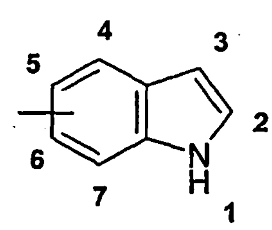

R étant éventuellement substitué en position 1, 2 et/ou 3 par un ou plusieurs groupes choisis parmi les groupes $C_1$-$C_6$-alkyle et $C_1$-$C_6$-fluoroalkyle ;

R étant éventuellement substitué en position 4, 5, 6 et/ou 7 par un ou plusieurs groupes choisis parmi les atomes d'halogène, les groupes $C_1$-$C_6$-alkyle, $C_1$-$C_6$-fluoroalkyle, $C_1$-$C_6$-alcoxy, $C_1$-$C_6$-fluoroalcoxy ;

Y représente un atome d'hydrogène ou un groupe $C_1$-$C_6$-alkyle;

n est égal à 0, 1, 2 ou 3 ;

$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkyle, ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe

étant éventuellement substitué par un groupe $C_1$-$C_6$-alkyle, $C_3$-$C_7$-cycloalkyle, $C_3$-$C_7$-cycloalkyle-$C_1$-$C_3$-alkyle ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle ou aryle.

Dans le cadre de la présente invention on entend par :

- $C_t$-$C_z$ où t et z peuvent prendre les valeurs de 1 à 6, une chaîne carbonée pouvant avoir de t à z atomes de carbone, par exemple $C_1$-$C_3$ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxy : un radical -O-alkyle où le groupe alkyle est tel que précédemment défini ;
- un fluoroalcoxy : un groupe alcoxy dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphtyle ;
- un atome d'halogène: un fluor, un chlore, un brome ou un iode.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Parmi les composés de formule (I) objets de l'invention, un premier sous-groupe de composés est constitué par les composés pour lesquels :

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène, plus particulièrement un fluor, un brome ou un chlore, ou un groupe $C_1$-$C_6$-alkyle, plus particulièrement un méthyle, un propyle, un isopropyle, un secbutyle, un tertiobutyle, un pentyle, $C_3$-$C_7$-cycloalkyle, plus particulièrement un cyclopentyle ou un cyclohexyle, $C_1$-$C_6$-fluoroalkyle, plus particulièrement un $CF_3$, $C_1$-$C_6$-alcoxy, plus particulièrement un méthoxy ou un éthoxy, $C_1$-$C_6$-fluoroalcoxy, plus particulièrement un $OCF_3$, nitro, $NR_1R_2$, $C_1$-$C_6$-thioalkyle, plus particulièrement un thiométhyle, -S(O)-$C_1$-$C_6$-alkyle, -$S(O)_2$-$C_1$-$C_6$-alkyle, plus particulièrement un -$S(O)_2$-$CH_3$, ou aryle, plus particulièrement phényle ; et/ou

$X_5$ représente un atome d'hydrogène ou un groupe $C_1$-$C_6$-alkyle, plus particulièrement un méthyle ; et/ou

R représente un groupe 4-, 5-, 6- ou 7-indolyle,

R =

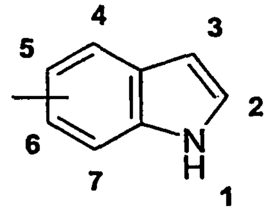

R étant éventuellement substitué en position 1, 2 et/ou 3 par un ou plusieurs groupes $C_1$-$C_6$-alkyle, plus particulièrement méthyle ou isopropyle ; et/ou

Y représente un atome d'hydrogène ; et/ou

n est égal à 0, 1, 2 ou 3 ;

$R_1$ et $R_2$ représentent, indépendamment l'un de l'autre, un atome d'hydrogène.

Parmi les composés de formule (I) objets de l'invention, un second sous-groupe de composés est constitué par les composés pour lesquels :

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène, plus particulièrement un fluor, un brome ou un chlore, ou un groupe $C_1$-$C_6$-alkyle, plus particulièrement un méthyle, un propyle, un isopropyle, un secbutyle, un tertiobutyle, un pentyle, $C_3$-$C_7$-cycloalkyle, plus particulièrement un cyclopentyle ou un cyclohexyle, $C_1$-$C_6$-fluoroalkyle, plus particulièrement un $CF_3$, $C_1$-$C_6$-alcoxy, plus particulièrement un méthoxy ou un éthoxy, $C_1$-$C_6$-fluoroalcoxy, plus particulièrement un $OCF_3$, nitro, $C_1$-$C_6$-thioalkyle, plus particulièrement un thiométhyle, -S(O)-$C_1$-$C_6$-alkyle, -$S(O)_2$-$C_1$-$C_6$-alkyle, plus particulièrement un -$S(O)_2$-$CH_3$, ou aryle, plus particulièrement phényle ; et/ou

$X_5$ représente un atome d'hydrogène ou un groupe $C_1$-$C_6$-alkyle, plus particulièrement un méthyle ; et/ou

R représente un groupe 4-, 5-, 6- ou 7-indolyle,

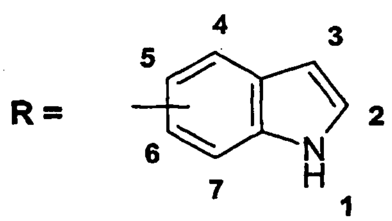

R étant éventuellement substitué en position 1, 2 et/ou 3 par un ou plusieurs groupes $C_1$-$C_6$-alkyle, plus particulièrement méthyle ; et/ou

Y représente un atome d'hydrogène ; et/ou

n est égal à 0, 1, 2 ou 3.

Parmi les composés de formule (I) objets de l'invention, un troisième sous-groupe de composés est constitué par les composés pour lesquels :

$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_1$-$C_6$-fluoroalkyle, $C_1$-$C_6$-alcoxy, $C_1$-$C_6$-fluoroalcoxy, cyano, C(O)$NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle ;

$X_5$ représente un atome d'hydrogène ou d'halogène ou un groupe $C_1$-$C_6$-alkyle, $C_1$-$C_6$-fluoroalkyle;

R représente un groupe 4-, 5-, 6- ou 7-indolyle,

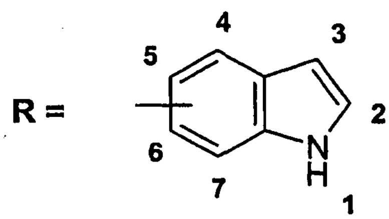

R étant éventuellement substitué en position 1, 2 et/ou 3 par un ou plusieurs groupes choisis parmi les groupes $C_1$-$C_6$-alkyle et $C_1$-$C_6$-fluoroalkyle ;

R étant éventuellement substitué en position 4, 5, 6 et/ou 7 par un ou plusieurs groupes choisis parmi les atomes d'halogène, les groupes $C_1$-$C_6$-alkyle, $C_1$-$C_6$-fluoroalkyle, $C_1$-$C_6$-alcoxy, $C_1$-$C_6$-fluoroalcoxy ;

Y représente un atome d'hydrogène ou un groupe $C_1$-$C_6$-alkyle ;

n est égal à 0, 1, 2 ou 3 ;

$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkyle, ou aryle ; ou $R_1$ et

$R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkyle ou aryle ;

$R_3$ et $R_4$ représentent, indépendamment l'un de l'autre, un atome d'hydrogène ou un groupe $C_1$-$C_6$-alkyle ou aryle ;

$R_5$ représente un groupe $C_1$-$C_6$-alkyle ou aryle ;

avec la condition que

quand $Z_1$, $Z_2$, $Z_3$, $Z_4$ et $Z_5$ représentent simultanément des atomes d'hydrogène
alors n est égal à 2 ou 3.

Parmi les composés de formule (I) objets de l'invention, un quatrième sous-groupe de composés est constitué par les composés pour lesquels :

R représente un groupe indol-5-yle

R =

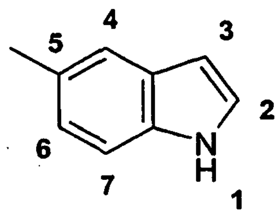

R étant éventuellement substitué en position 1, 2 et/ou 3 par un ou plusieurs groupes choisis parmi les groupes $C_1$-$C_6$-alkyle et $C_1$-$C_6$-fluoroalkyle ;
R étant éventuellement substitué en position 4, 6 et/ou 7 par un ou plusieurs groupes choisis parmi les atomes d'halogène, les groupes $C_1$-$C_6$-alkyle, $C_1$-$C_6$-fluoroalkyle, $C_1$-$C_6$-alcoxy, $C_1$-$C_6$-fluoroalcoxy ;
$X_1$, $X_2$, $X_3$, $X_4$, $X_5$, $Z_1$, $Z_2$, $Z_3$, $Z_4$, $Z_5$ Y, n, $R_1$, $R_2$, $R_3$, $R_4$ et $R_5$ étant tels que définis dans la formule générale (I) ci-dessus ou tels que définis dans le premier, le second ou le troisième sous-groupe ci-dessus.

Parmi les composés de formule (I) objets de l'invention, un cinquième sous-groupe de composés est constitué par les composés pour lesquels :

$X_2$ et/ou $X_3$ sont différents d'un atome d'hydrogène ;
$X_1$, $X_3$, $X_4$, $X_5$, $Z_1$, $Z_2$, $Z_3$, $Z_4$, $Z_5$, R, Y, n, $R_1$, $R_2$, $R_3$, $R_4$ et $R_5$ étant tels que définis dans la formule générale (I) ci-dessus ou tels que définis dans le premier, le second, le troisième ou le quatrième sous-groupe ci-dessus.

Parmi les composés de formule (I) objets de l'invention, un sixième sous-groupe de composés est constitué par les composés pour lesquels :

$X_5$ représente un atome d'hydrogène ;
$X_1$, $X_2$, $X_3$, $X_4$, $Z_1$, $Z_2$, $Z_3$, $Z_4$, $Z_5$, R, Y, n, $R_1$, $R_2$, $R_3$, $R_4$, et $R_5$ étant tels que définis dans la fomule générale (I) ci-dessus ou tels que définis dans le premier, le second, le troisième, le quatrième ou le cinquième sous-groupe ci-dessus.

Parmi les composés de formule (I) objets de l'invention, un septième sous-groupe de composés est constitué par les composés pour lesquels :

Y représente un atome d'hydrogène ;
$X_1$, $X_2$, $X_3$, $X_4$, $X_5$, $Z_1$, $Z_2$, $Z_3$, $Z_4$, $Z_5$, R; n, $R_1$, $R_2$, $R_3$, $R_4$ et $R_5$ étant tels que définis dans la formule générale (I) ci-dessus ou tels que définis dans le premier, le second, le troisième, le quatrième, le cinquième ou le sixième sous-groupe ci-dessus.

Conformément à l'invention on peut préparer les composés de formule générale (I) selon le procédé illustré par le schéma 1 qui suit.
Selon le schéma 1, les composés de formule générale (IV) peuvent être obtenus par réaction d'un composé de formule générale (II) dans laquelle $X_1$, $X_2$, $X_3$, $X_4$, $X_5$ sont tels que définis dans la formule générale (I) ci-dessus et A représente un groupe $C_1$-$C_6$-alcoxy ou hydroxy, avec un composé de formule générale (III), dans laquelle $Z_1$, $Z_2$, $Z_3$, $Z_4$, $Z_5$ et n sont tels que définis dans la formule générale (I) ci-dessus et R' représente un atome de brome, d'iode, un groupe tosylate ou tout autre groupe équivalent.

Schéma 1

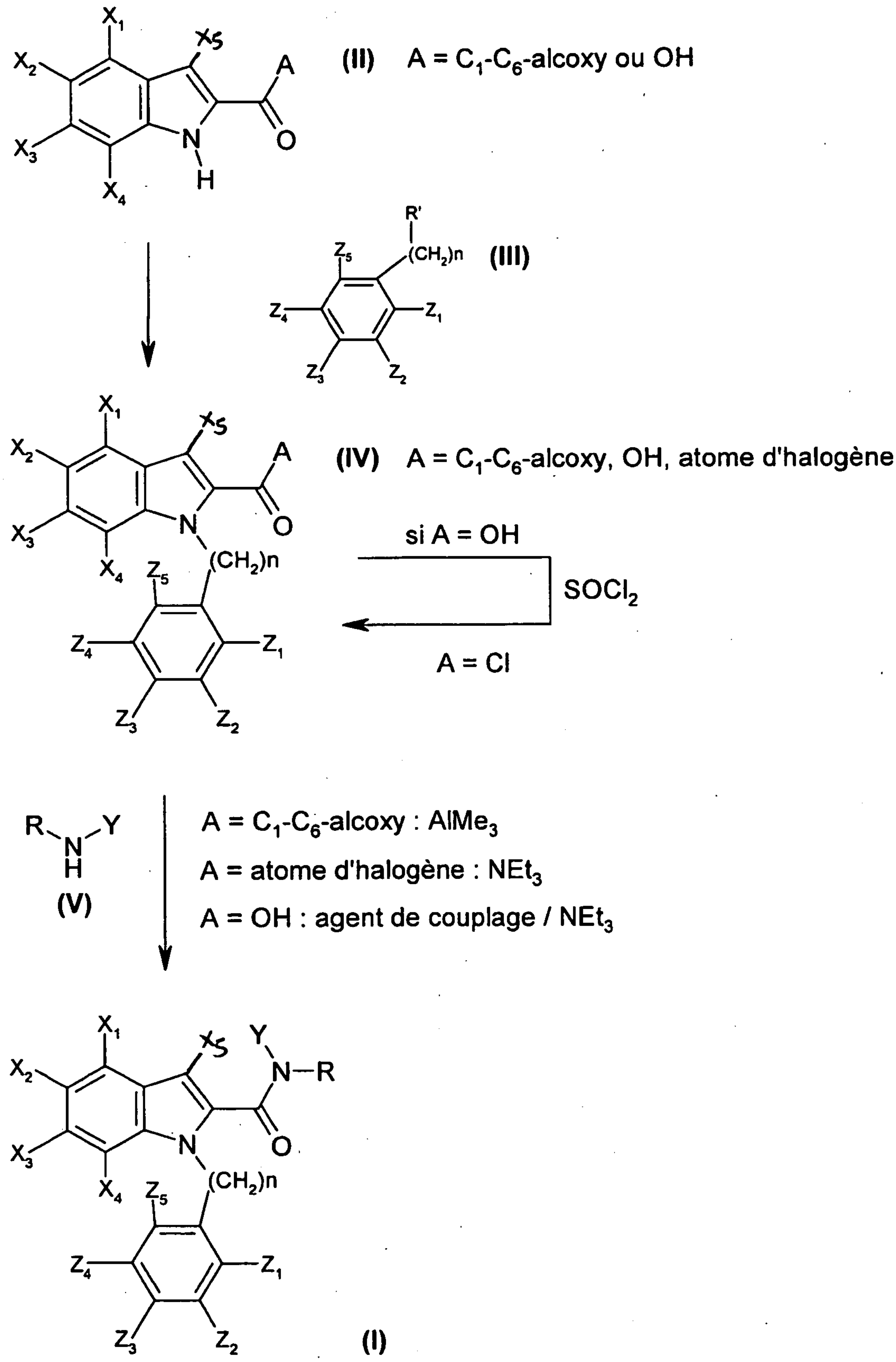


Lorsque n = 1, 2 ou 3, le composé de formule générale (III) peut être un halogénure d'alkyle, tel qu'un bromure

de benzyle (n = 1 : Kolasa T., Bioorg.Med.Chem. 1997, 5 (3) 507) ou un iodure de phénéthyle (n = 2 : Abramovitch R., Synth. Commun., 1995, 25 (1), 1), et la réaction peut être réalisée en présence d'une base telle que l'hydrure de sodium ou le carbonate de potassium, dans un solvant polaire tel que le diméthylformamide, le diméthylsulfoxyde ou l'acétone. Lorsque n = 0, le composé de formule générale (III) est un iodure ou un bromure d'aryle et la réaction peut être réalisée à une température comprise entre 80°C et 250°C, en présence d'un catalyseur à base de cuivre tel que le bromure de cuivre ou l'oxyde de cuivre ainsi que d'une base telle que le carbonate de potassium (Murakami Y., Chem.Pharm.Bull., 1995, 43 (8), 1281). On peut également utiliser les conditions plus douces, décrites dans S.L. Buchwald, J.Am.Chem.Soc. 2002, 124, 11684.

Alternativement, les composés de formule générale (IV), dans laquelle n = 0, peuvent être obtenus par réaction du composé de formule générale (II) avec un composé de formule générale (III) de type acide boronique (n = 0, R' = B$(OH)_2$), en présence d'une base telle que la triéthylamine ou la pyridine ainsi que de diacétate de cuivre, par analogie avec des protocoles décrits dans W.W.K.R. Mederski, Tetrahedron, 1999, 55, 12757.

Les composés de formule générale (II) sont disponibles dans le commerce ou préparés selon de nombreux procédés décrits dans la littérature (D. Knittel Synthesis 1985, 2, 186 et T.M. Williams J.Med.Chem. 1993, 36 (9), 1291 par exemple).

Dans le cas des indoles de formule générale (IV), dans laquelle A représente un groupe $C_1$-$C_6$-alcoxy, le composé de formule générale (I) est obtenu par réaction d'un composé de formule générale (IV), tel qu'obtenu ci-dessus, avec un amidure du composé de formule générale (V), dans laquelle R et Y sont tels que définis dans la formule générale (I) ci-dessus, au reflux d'un solvant tel que le toluène. L'amidure du composé de formule générale (V) est préparé par action préalable du triméthylaluminium sur les aminoindoles de formule générale (V).

Dans le cas des indoles de formule générale (IV), dans laquelle A représente un groupe hydroxy, la fonction acide carboxylique peut préalablement être transformée en halogénure d'acide tel qu'un chlorure d'acide par action du chlorure de thionyle, au reflux d'un solvant tel que le dichlorométhane ou le dichloroéthane. Le composé de formule générale (I) est alors obtenu par réaction du composé de formule générale (IV), dans laquelle A représente un atome de chlore, avec l'aminoindole de formule générale (V), en présence d'une base telle que la triéthylamine.

Alternativement, l'indole de formule générale (IV), dans laquelle A représente un groupe hydroxy, peut être couplé à l'aminoindole de formule générale (V) en présence d'un agent de couplage tel qu'un dialkylcarbodiimide, l'hexafluorophosphate de benzotriazole-1-yl-oxy-trispyrrolidinophosphonium, le diéthylcyanophosphonate ou tout autre agent de couplage connu de l'homme de l'art, en présence d'une base comme la triéthylamine, dans un solvant tel que le diméthylformamide.

Les aminoindoles de formule générale (V) sont préparés selon des procédés décrits dans la littérature tels que dans I.T. Forbes, J.Med.Chem. 1993, 36 (8), 1104 (Y = H), I.T. Forbes, WO9205170 (Y = alkyle).

Dans le schéma 1, les composés de formule (II), (III) et (V) et les autres réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les composés de formules générales (II), (IV) et (I), dans lesquelles $X_1$, $X_2$, $X_3$, $X_4$, $X_5$, $Z_1$, $Z_2$, $Z_3$, $Z_4$ et/ou $Z_5$ représentent un groupe cyano ou un aryle, peuvent être obtenus par une réaction de couplage, catalysée par un métal tel que le palladium, réalisée sur les composés de formules générales (II), (IV) ou (I) correspondants, dans lesquelles $X_1$, $X_2$, $X_3$, $X_4$, $X_5$, $Z_1$, $Z_2$, $Z_3$, $Z_4$ et/ou $Z_5$ représente un atome de brome.

Les composés de formules générales (II), (IV) et (I), dans lesquelles $X_1$, $X_2$, $X_3$, $X_4$, $X_5$, $Z_1$, $Z_2$, $Z_3$, $Z_4$ et/ou $Z_5$ représentent un groupe $C(O)NR_1R_2$, peuvent être obtenus à partir des composés de formules générales (II), (IV) ou (I) correspondants, dans lesquelles $X_1$, $X_2$, $X_3$, $X_4$, $X_5$, $Z_1$, $Z_2$, $Z_3$, $Z_4$ et/ou $Z_5$ représente groupe cyano, selon des méthodes qui sont décrites dans la littérature ou qui sont connues de l'homme du métier.

Les composés de formules générales (II), (IV) et (I), dans lesquelles $X_1$, $X_2$, $X_3$, $X_4$, $X_5$, $Z_1$, $Z_2$, $Z_3$, $Z_4$ et/ou $Z_5$ représentent groupe S(O)-alkyle ou $S(O)_2$-alkyle, peuvent être obtenus par oxydation des composés de formules générales (II), (IV) ou (I) correspondants, dans lesquelles $X_1$, $X_2$, $X_3$, $X_4$, $X_5$, $Z_1$, $Z_2$, $Z_3$, $Z_4$ et/ou $Z_5$ représente un groupe $C_1$-$C_6$-thioalkyle, selon des méthodes qui sont décrites dans la littérature ou qui sont connues de l'homme du métier.

Les composés de formules générales (II), (IV) et (I), dans lesquelles $X_1$, $X_2$, $X_3$, $X_4$, $X_5$, $Z_1$, $Z_2$, $Z_3$, $Z_4$ et/ou $Z_5$ représentent groupe $NR_1R_2$, $NR_3COR_4$ ou $NR_3SO_2R_4$, peuvent être obtenus à partir des composés de formules générales (II), (IV) ou (I) correspondants, dans lesquelles $X_1$, $X_2$, $X_3$, $X_4$, $X_5$, $Z_1$, $Z_2$, $Z_3$, $Z_4$ et/ou $Z_5$ représente un groupe nitro, par exemple par réduction, puis acylation ou sulfonylation, selon des méthodes qui sont décrites dans la littérature ou qui sont connues de l'homme du métier.

Les composés de formules générales (II), (IV) et (1), dans lesquelles $X_1$, $X_2$, $X_3$, $X_4$, $X_5$, $Z_1$, $Z_2$, $Z_3$, $Z_4$ et/ou $Z_5$ représentent groupe $SO_2NR_1R_2$ peuvent être obtenus par une méthode analogue à celle décrite dans Pharmazie 1990, 45, 346, ou selon des méthodes qui sont décrites dans la littérature ou qui sont connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. et R.M.N. confirment les structures des composés obtenus.

Sauf indication, les réactifs chimiques utilisés dans les exemples sont tous disponibles dans le commerce.

**Exemple 1 (Composé N°1)**

*N*-(1-méthyl-1*H*-indol-5-yl)-1-(3-trifluorométhylbenzyl)-1*H*-indole-2-carboxamide

1.1 1-(3-trifluorométhylbenzyl)-1*H*-indole-2-carboxylate d'éthyle

On agite une suspension de 0,492 g (2,6 mmoles) de 1*H*-indole-2-carboxylate d'éthyle, de 0,683 g (2,86 mmoles) de bromure de 3-trifluorométhylbenzyle et de 0,898 g (6,5 mmoles) de carbonate de potassium dans 50 mL de diméthylformamide pendant 24 heures à 60°C. On refroidit le mélange réactionnel, on le verse dans un mélange d'eau glacée et d'acétate d'éthyle. Après décantation, on sépare la phase organique puis on la lave avec deux fois 50 mL d'eau puis avec 50 mL d'une solution saturée en chlorure de sodium. On sèche la solution sur sulfate de magnésium, on la filtre puis on concentre le filtrat sous pression réduite. On obtient 0,8 g d'une huile utilisée telle quelle dans l'étape suivante.

1.2 *N*-(1-méthyl-1*H*-indol-5-yl)-1-(3-trifluorométhylbenzyl)-1*H*-indole-2-carboxamide (composé n°1)

On ajoute goutte à goutte à 0°C, une solution de 0,231 g (1,58 mmole) de 1-méthyl-1*H*-5-aminoindole (I.T. Forbes, J.Med.Chem. 1993, 36 (8), 1104) dans 15 mL de toluène à une solution de 0,93 mL (1,87 mmole) de triméthylaluminium (2M dans le toluène) dans 6 mL de toluène. Après 15 minutes d'agitation, on additionne 0,5 g (1,44 mmole) de 1-(3-trifluorométhylbenzyl)-1*H*-indole-2-carboxylate d'éthyle, obtenu à l'étape 1.1. On chauffe le mélange pendant 4 heures à 50°C. On hydrolyse ensuite le mélange réactionnel par ajout de 10 mL d'eau puis on le reprend par 100 mL d'acétate d'éthyle. On lave la phase organique par 100 mL d'acide chlorhydrique 1N, avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le résidu par chromatographie sur colonne de silice en éluant avec un mélange de cyclohexane et de dichlorométhane, puis on le recristallise dans l'isopropanol. On obtient ainsi 0,33 g de produit.
Point de fusion : 189 - 190 °C
R.M.N. $^1H$ (DMSO $D_6$) : $\delta$ (ppm) : 3,75 (s, 3H), 5,93 (s, 2H), 6,38 (d, 1H), 7,4 (m, 11H), 7,71 (d, 1 H), 7,96 (s, 1 H).

**Exemple 2 (composé n°2)**

N-(1-méthyl-1*H*-indol-5-yl)-5-méthoxy-1-(3-trifluorométhylbenzyl)-1*H*-indole-2-carboxamide

2.1 5-méthoxy-1*H*-indole-2-carboxylate d'éthyle

A une solution de 1 g (5,23 mmoles) d'acide 5-méthoxy-1*H*-indole-2-carboxylique dans 52 mL d'éthanol, on additionne goutte à goutte 1,91 mL (26,15 mmoles) de chlorure de thionyle, sous agitation à 0°C. On chauffe le mélange réactionnel au reflux pendant 2 heures puis on le refroidit et on le concentre sous pression réduite. On reprend le résidu avec 100 mL d'acétate d'éthyle et on lave cette solution avec deux fois 50 mL d'eau puis avec 50 mL d'une solution saturée en chlorure de sodium. On sèche la solution sur sulfate de magnésium, on la filtre puis on concentre le filtrat sous pression réduite. On obtient 1,2 g de produit utilisé tel quel dans l'étape suivante.

2.2 5-méthoxy-1-(3-trifluorométhylbenzyl)-1*H*-indole-2-carboxylate d'éthyle

A une suspension de 0,306 g d'hydrure de sodium dans 10 mL de diméthylformamide, on ajoute goutte à goutte une solution de 1,2 g (5,47 mmoles) de 5-méthoxy-1*H*-indole-2-carboxylate d'éthyle, obtenu à l'étape 2.1, dans 50 mL de diméthylformamide. On agite le mélange pendant 1 heure à température ambiante puis on ajoute 1,01 mL (6,57 mmoles) de bromure de 3-trifluorométhylbenzyle et on maintient l'agitation pendant 4 heures supplémentaires. On verse le mélange réactionnel sur 200 mL d'eau glacée et 100 mL d'acétate d'éthyle. Après décantation, on sépare la phase organique puis on la lave avec trois fois 50 mL d'eau puis avec 50 mL d'une solution saturée en chlorure de sodium. On sèche la solution sur sulfate de magnésium, on la filtre puis on concentre le filtrat sous pression réduite. On obtient 2 g de produit utilisé tel quel dans l'étape suivante.

2.3 *N*-(1-méthyl-1*H*-indol-5-yl)-5-méthoxy-1-(3-trifluorométhylbenzyl)-1*H*-indole-2-carboxamide (composé n°2)

On ajoute, goutte à goutte à 0°C, une solution de 0,278 g (1,91 mmole) de 1-méthyl-1*H*-5-aminoindole (I.T.

Forbes, J.Med.Chem. 1993, 36 (8), 1104) dans 15 mL de toluène à une solution de 1,59 mL (3,18 mmoles) de triméthylaluminium (2M dans le toluène) dans 10 mL de toluène. Après 15 minutes d'agitation, on additionne 0,6 g (1,59 mmole) de 1-(3-trifluorométhylbenzyl)-5-méthoxy-1*H*-indole-2-carboxylate d'éthyle, obtenu à l'étape 2.2. On chauffe le mélange pendant 4 heures à 50˚C. On hydrolyse le mélange réactionnel par ajout de 10 mL d'eau puis on le reprend par 100 mL d'acétate d'éthyle. On lave la phase organique par 100 mL d'acide chlorhydrique 1N, avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le produit résultant par chromatographie sur colonne de silice en éluant avec un mélange de cyclohexane et d'acétate d'éthyle, puis on le recristallise dans l'isopropanol. On obtient 0,55 g de produit.
Point de fusion : 176 - 177 ˚C
R.M.N. $^1$H (DMSO $D_6$) : δ (ppm) : 3,8 (s, 3H), 3,89 (s, 3H), 5,9 (s, 2H), 6,49 (d, 1 H), 7,2 (m, 8H), 7,48 (m, 2H), 7,9 (m, 2H).

**<u>Exemple 3</u> (composé n˚3)**

*N*-(1-méthyl-1*H*-indol-5-yl)-5-fluoro-1-(3-fluorobenzyl)-1*H*-indole-2-carboxamide

3.1 5-fluoro-1-(3-fluorobenzyl)-1*H*-indole-2-carboxylate d'éthyle

On agite une suspension de 0,207 g (1 mmole) de 5-fluoro-1*H*-indole-2-carboxylate d'éthyle, 0,173 g (1,2 mmole) de chlorure de 3-fluorobenzyle et 0,276 g (2 mmoles) de carbonate de potassium dans 10 mL de diméthylformamide pendant 24 heures à 60˚C. On refroidit ensuite le mélange réactionnel, on le verse dans un mélange d'eau glacée et d'acétate d'éthyle. Après décantation, on sépare la phase organique puis on la lave avec deux fois 50 mL d'eau puis avec 50 mL d'une solution saturée en chlorure de sodium. On sèche la solution sur sulfate de magnésium, on la filtre puis on concentre le filtrat sous pression réduite. On obtient 0,195 g d'une huile utilisée telle quelle dans l'étape suivante.

3.2 *N*-(1-méthyl-1*H*-indol-5-yl)-5-fluoro-1-(3-fluorobenzyl)-1*H*-indole-2-carboxamide (composé n˚3)

On ajoute, goutte à goutte à 0˚C, une solution de 0,146 g (0,7 mmole) de 1-méthyl-1*H*-5-aminoindole (I.T. Forbes, J.Med.Chem. 1993, 36 (8), 1104) dans 15 mL de toluène à une solution de 0,7 mL (1,4 mmole) de triméthylaluminium (2M dans le toluène) dans 3 mL de toluène. Après 15 minutes d'agitation, on additionne 0,195 g (0,62 mole) de 5-fluoro-1-(3-fluorobenzyl)-1*H*-indole-2-carboxylate d'éthyle, obtenu à l'étape 3.1. On chauffe le mélange pendant 4 heures à 50˚C. On hydrolyse le mélange réactionnel par ajout de 10 mL d'eau puis on le reprend par 100 mL d'acétate d'éthyle. On lave la phase organique par 100 mL d'acide chlorhydrique 1 N, avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le résidu par chromatographie sur colonne de silice en éluant avec un mélange de cyclohexane et de dichlorométhane. On obtient 0,152 g de produit.
Point de fusion = 187 - 189 ˚C
R.M.N. $^1$H (DMSO $D_6$) : δ (ppm) : 3,77 (s, 3H), 5,87 (s, 2H), 6,38 (d, 1H), 7 (m, 4H), 7,32 (m, 7H), 7,98 (s, 1 H).

**<u>Exemple 4</u> (composé n˚30)**

*N*-(1-méthyl-1*H*-indol-5-yl)-1-(4-isopropylphényl)-1*H*-indole-2-carboxamide

4.1 Acide 1-(4-isopropylphényl)-1*H*-indole-2-carboxylique

On agite une suspension, dans 200 mL de diméthylformamide, de 128,8 g (0,8 mole) d'acide 1*H*-indole-2-carboxylique, de 159,2 g (0,8 mole) de 4-bromocumène, de 111,6 g (0,808 mole) de carbonate de potassium et de 8 g (0,1 mole) d'oxyde de cuivre, à reflux pendant 24 heures. Après refroidissement, on ajoute 6 L d'eau à la suspension beige obtenue. On filtre la suspension, puis on reprend l'insoluble par 1 L d'une solution d'acide chlorhydrique 5N. On extrait ce mélange par 500 mL de dichlorométhane. On lave la phase organique à l'eau, on la sèche sur sulfate de sodium puis on la concentre sous pression réduite. Après séchage sous pression réduite, on obtient 204,4 g d'un solide blanc utilisé tel quel dans l'étape suivante.
Point de fusion = 203 - 204 ˚C

4.2 Chlorure d'acide 1-(4-isopropylphényl)-1*H*-indole-2-carboxylique

On agite une solution de 111 mg (0,4 mmole) d'acide 1-(4-isopropylphényl)-1*H*-indole-2-carboxylique, obtenu

à l'étape 4.1, et de 90 microlitres (1,2 mmole) de chlorure de thionyle dans 2 mL de dichloroéthane, pendant 3 heures au reflux. On concentre le milieu réactionnel sous pression réduite. On obtient un résidu utilisé tel quel dans l'étape suivante.

4.3 *N*-(1-méthyl-1*H*-indol-5-yl)-1-(4-isopropylphényl)-1*H*-indole-2-carboxamide (composé n° 30)

On agite une solution de 119 mg (0,4 mmole) de chlorure d'acide 1-(4-isopropylphényl)-1*H*-indole-2-carboxylique, obtenu à l'étape 4.2, 70 mg (0,48 mmole) de 1-méthyl-1*H*-5-aminoindole et 110 microlitres (0,8 mmole) de triéthylamine dans 2 mL de tétrahydrofuranne, pendant 18 heures à température ambiante. On concentre le mélange réactionnel sous pression réduite, on le reprend avec 20 mL d'eau et 50 mL de dichlorométhane. On sépare la phase organique, on la lave avec 50 mL d'acide chlorhydrique 1N, on la sèche sur sulfate de magnésium puis on la concentre sous pression réduite. On purifie le résidu par chromatographie sur colonne de silice en éluant avec un mélange de cyclohexane et d'acétate d'éthyle. On obtient 0,133 g de produit.
Point de fusion : 178 - 179 °C
R.M.N. $^1H$ ($CDCl_3$) : $\delta$ (ppm) : 1,39 (d, 6H), 3,05 (sept., 1H), 3,8 (s, 3H), 6,4 (d, 1H), 7,29 (m, 11 H), 7,78 (m, 3H).

**Exemple 5 (composé n°4)**

*N*-(1-méthyl-1*H*-indol-5-yl)-1-(3-trifluorométhylphényl)-1*H*-indole-2-carboxamide

5.1 Acide 1-(3-trifluorométhylphényl)-1*H*-indole-2-carboxylique

Le composé peut être préparé selon une méthode analogue à celle décrite à l'étape 4.1 de l'exemple 4, en remplaçant le 4-bromocumène par le 3-bromo-$\alpha,\alpha,\alpha$-trifluorotoluène.

5.2 *N*-(1-méthyl-1*H*-indol-5-yl)-1-(3-trifluorométhylphényl)-1*H*-indole-2-carboxamide

On agite une solution de 2 g (6,55 mmoles) d'acide 1-(3-trifluorométhylphényl)-1*H*-indole-2-carboxylique (prépapré par analogie à la méthode décrite à l'étape 4.1 de l'exemple 4, 1,14 g (7,86 mmoles) de 1-méthyl-1*H*-5-aminoindole (I.T. Forbes, J.Med.Chem. 1993, 36 (8), 1104), 1,2 mL (7,86 mmoles) de diéthylcyanophosphonate et 2,03 mL (14,41 mmoles) de triéthylamine, dans 20 mL de diméthylformamide, pendant 18 heures à température ambiante. On concentre le mélange réactionnel sous pression réduite puis on le reprend par 50 mL d'eau. On extrait cette solution avec deux fois 50 mL de dichlorométhane. On réunit les phases organiques, on les sèche sur sulfate de sodium puis on les concentre sous pression réduite. On purifie le résidu obtenu par chromatographie sur colonne de silice en éluant avec un mélange de cyclohexane et d'acétate d'éthyle.
On isole 1,97g de produit.
Point de fusion : 225 - 226 °C
R.M.N. $^1H$ (DMSO $D_6$) : $\delta$ (ppm) : 3,79 (s, 3H), 6,41 (d, 1H), 7,05 (d, 1H), 7,28 (m, 3H), 7,7 (m, 7H).

**Exemple 6 (composé n°41)**

*N*-(1-méthyl-1*H*-indol-5-yl)-1-(3-isopropylphényl)-5-trifluorométhyloxy-1*H*-indole-2-carboxamide

6.1 1-(3-isopropylphényl)-5-trifluorométhyloxy-1*H*-indole-2-carboxylate d'éthyle

On agite un mélange de 0,2 g (0,73 mmole) de 5-trifluorométhyloxy-1*H*-indole-2-carboxylate d'éthyle, de 0,24 g (1,46 mmole) d'acide 3-isopropylphénylboronique, de 0,2 g (1,1 mmole) de diacétate de cuivre et de 0,12 mL (1,46 mmole) de pyridine dans 5 mL de dichlorométhane, en présence de tamis moléculaire 4 A, pendant 4 jours à température ambiante. On verse le mélange sur 100 mL d'eau et 50 mL de dichlorométhane. On sépare la phase organique, on la lave avec de l'acide chlorhydrique 1 N, on la sèche sur sulfate de magnésium puis on la concentre sous pression réduite. On purifie le résidu par chromatographie sur colonne de silice en éluant avec un mélange de cyclohexane et d'acétate d'éthyle. On obtient 0,1 g de produit utilisé tel quel dans l'étape suivante.

6.2 *N*-(1-méthyl-1*H*-indol-5-yl)-1-(3-isopropylphényl)-5-trifluorométhyloxy-1*H*-indole-2-carboxamide (composé n°41)

On ajoute, goutte à goutte à 0°C, une solution de 0,0493 g (0,34 mmole) de 1-méthyl-5-amino-1*H*-indole (I.T. Forbes, J.Med.Chem. 1993, 36 (8), 1104) dans 5 mL de toluène à une solution de 0,28 mL (0,56 mmole) de triméthylaluminium (2M dans le toluène) dans 2 mL de toluène. Après 15 minutes d'agitation, on additionne 0,1 g (0,28 mmole)

de 1-(3-isopropylphényl)-5-trifluorométhyloxy-1*H*-indole-2-carboxylate d'éthyle, obtenu à l'étape 6.1. On chauffe le mélange pendant 4 heures à 50˚C. On hydrolyse le mélange réactionnel par ajout de 10 mL d'eau puis on le reprend par 100 mL d'acétate d'éthyle. On lave la phase organique par 100 mL d'acide chlorhydrique 1 N, avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le résidu par chromatographie sur colonne de silice en éluant avec un mélange de cyclohexane et d'acétate d'éthyle, puis on le recristallise dans l'isopropanol. On obtient 0,136 g de produit.
Point de fusion : 164 - 165 ˚C
R.M.N. $^1$H (DMSO $D_6$) : δ (ppm) : 1,22 (dxs, 6H), 2,98 (m, 1 H), 3,79 (s, 3H), 6,38 (d, 1H), 7,4 (m, 11H), 7,9 (m, 2H).

**<u>Exemple 7</u> (composé n˚70)**

*N*-(1*H*-indol-5-yl)-5-fluoro-1-(3-fluorobenzyl)-1*H*-indole-2-carboxamide

On ajoute, goutte à goutte à 0˚C, une solution de 0,46 g (3,49 mmoles) de 5-amino-1*H*-indole dans 50 mL de toluène à une solution de 4,76 mL (9.51 mmoles) de triméthylaluminium (2M dans le toluène) dans 10 mL de toluène. Après 15 minutes d'agitation, on additionne 1 g (3,17 mmoles) du 5-fluoro-1-(3-fluorobenzyl)-1*H*-indole-2-carboxylate d'éthyle, obtenu à l'étape 3.1 de l'exemple 3. On chauffe le mélange pendant 4 heures à 50˚C. On hydrolyse le mélange réactionnel par ajout de 10 mL d'eau puis on le reprend par 100 mL d'acétate d'éthyle. On lave la phase organique par 100 mL d'acide chlorhydrique 1N, avec deux fois 50 mL d'eau puis avec 50 mL d'une solution saturée en chlorure de sodium. On sèche la solution sur sulfate de magnésium, on la filtre puis on concentre le filtrat sous pression réduite. On purifie le résidu par chromatographie sur colonne de silice en éluant avec un mélange de cyclohexane et de dichlorométhane. On obtient 0,7 g de produit.
Point de fusion = 158 - 163 ˚C
R.M.N. $^1$H (DMSO $D_6$) : δ (ppm) : 5,87 (s, 2H), 6,38 (m, 1 H), 6.9 (m, 2H), 7,1 (m, 2H), 7,31 (m, 5H), 7,51 (m, 2H), 7,92 (s, 1H), 10,26 (s, 1H), 10,98 (s, 1 H).
Le tableau 1 qui suit illustre les structures chimiques et les propriétés physiques de quelques composés de formule générale (I) selon l'invention. Dans ce tableau :

- la colonne "PF" renseigne les points de fusion des produits en degrés Celsius (˚C). Lorsque les produits ont été isolés sous la forme de solide amorphe ou d'huile, ils sont caractérisés dans cette colonne par leur masse ($[MH]^+$) ;
- Me, MeO, EtO, n-Pr, i-Pr, s-Bu, t-Bu représentent respectivement des groupes méthyle, méthoxy, éthoxy, propyle, isopropyle, secbutyle, terbutyle.

**Tableau 1**

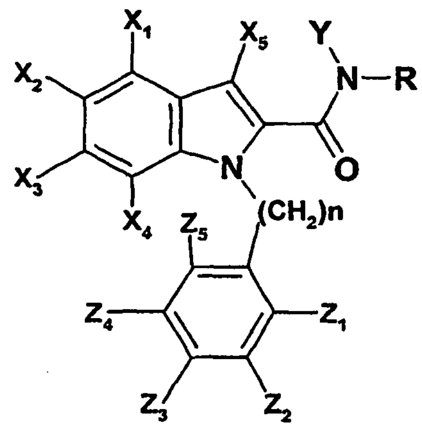

(I)

| N° | $X_1, X_2, X_3, X_4, X_5$ | R | Y | n | $Z_1$ | $Z_2$ | $Z_3$ | $Z_4$ | $Z_5$ | PF (°C) |
|---|---|---|---|---|---|---|---|---|---|---|
| 1 | H, H, H, H, H | 1-Méthylindol-5-yl | H | 1 | H | $CF_3$ | H | H | H | 189 - 190 |
| 2 | H, MEO, H, H, H | 1-Méthylindol-5-yl | H | 1 | H | $CF_3$ | H | H | H | 176 - 177 |
| 3 | H, F, H, H, H | 1-Méthylindol-5-yl | H | 1 | H | F | H | H | H | 187 - 189 |
| 4 | H, H, H, H, H | 1-Méthylindol-5-yl | H | 0 | H | $CF_3$ | H | H | H | 225 - 226 |
| 5 | H, H, H, H, H | 1-Méthylindol-5-yl | H | 0 | H | Me | H | Me | H | 142 - 144 |
| 6 | H, Me, H, H, H | 1-Méthylindol-5-yl | H | 1 | H | $CF_3$ | H | H | H | 195 - 196 |
| 7 | H, H, H, H, H | 1-Méthylindol-5-yl | H | 0 | H | H | H | H | H | 182 - 184 |
| 8 | H, H, HeO, H, | 1-Méthylindol-5-yl | H | 1 | H | $CF_3$ | H | H | H | 160 - 161 |
| 9 | H, Cl, H, H, H | 1-Méthylindol-5-yl | H | 1 | H | $CF_3$ | H | H | H | 205 - 206 |
| 10 | MeO, H, H, H, H | 1-Méthylindol-5-yl | H | 1 | H | $CF_3$ | H | H | H | 215 - 217 |
| 11 | H, F, H, H, H | 1-Méthylindol-5-yl | H | 1 | H | $CF_3$ | H | H | H | 188 - 191 |
| 12 | H, F, H, H, H | 1-Méthylindol-5-yl | H | 1 | H | H | $CF_3$ | H | H | 220- 221 |
| 13 | H, F, H, H, H | 1-Méthylindol-5-yl | H | 1 | H | $CF_3$ | H | H | Cl | 199 - 200 |

(suite)

| N° | $X_1, X_2, X_3, X_4, X_5$ | R | Y | n | $Z_1$ | $Z_2$ | $Z_3$ | $Z_4$ | $Z_5$ | PF (°C) |
|---|---|---|---|---|---|---|---|---|---|---|
| 14 | H, F, H, H, H | 1-Méthylindol-5-yl | H | 1 | H | Me | H | H | H | 161 - 163 |
| 15 | H, F, H, H, H | 1-Méthylindol-5-yl | H | 1 | H | MeO | H | H | H | $[MH]^+$ : 428 |
| 16 | H, F, H, H, H | 1-Méthylindol-5-yl | H | 1 | H | $CF_3O$ | H | H | H | 173 - 174 |
| 17 | H, F, H, H, H | 1-Méthylindol-5-yl | H | 1 | H | H | t-Bu | H | H | 217 - 218 |
| 18 | H, F, H, H, H | 1-Méthylindol-5-yl | H | 1 | H | Cl | H | H | H | 171 - 172 |
| 19 | H, H, H, H, H | 1-Méthylindol-5-yl | H | 0 | H | H | F | H | H | 224 - 225 |
| 20 | H, H, H, H, H | 1-Méthylindol-5-yl | H | 0 | H | H | SMe | H | H | 73 - 74 |
| 21 | H, H, H, H, H | 1-Méthylindol-5-yl | H | 0 | H | H | s-Bu | H | H | 191 - 192 |
| 22 | H H, H, H | 1-Méthylindol-5-yl | H | 0 | H | H | H | H | H | 166 - 168 |
| 23 | H, H, Me, H, H | 1-Méthylindol-5-yl | H | 0 | H | H | i-Pr | H | H | 147 - 148 |
| 24 | H, H, H, H, H | 1-Méthylindol-5-yl | H | 0 | H | H | n-pentyl | H | H | $[MH]^+$ : 436 |
| 25 | H, H, H, H, H | 1-Méthylindol-5-yl | H | 0 | H | H | cyclopentyl | H | H | 221 - 222 |
| 26 | H, H, H, H, H | 1-Méthylindol-5-yl | H | 0 | H | H | Ph | H | H | 194 - 195 |
| 27 | H, H, H, H, H | 1-Méthylindol-5-yl | H | 0 | H | H | $CF_3$ | H | H | 233- 235 |
| 28 | H, H, H, H, H | 1-Méthylindol-5-yl | H | 0 | H | H | n-Pr | H | H | 144-146 |
| 29 | H, H, H, H, H | 1-Méthylindol-5-yl | H | 0 | H | Me | H | H | H | 86-88 |
| 30 | H, H, H, H, H | 1-Méthylindol-5-yl | H | 0 | H | H | i-Pr | H | H | 178 - 179 |
| 31 | H, H, H, H, H | 1-Méthylindol-5-yl | H | 0 | H | H | t-Bu | H | H | 169 - 170 |
| 32 | H, H, H, H, H | 1-Méthylindol-5-yl | H | 0 | H | H | cyclohexyle | H | H | 227 - 229 |
| 33 | H, H, H, H, H | 1-Méthylindol-5-yl | H | 0 | H | H | EtO | H | H | 94 - 95 |
| 34 | H, H, H, H, H | 1-Méthylindol-5-yl | H | 0 | H | H | Cl | H | H | $[MH]^+$ : 400 |
| 35 | H, F, H, H, H | 1-Méthylindol-5-yl | H | 1 | H | F | H | F | H | $[MH]^+$ : 434 |
| 36 | H, F, H, H, H | 1-Méthylindol-5-yl | H | 1 | F | H | H | H | H | 204 - 206 |
| 37 | H, F, H, H, H | 1-Méthylindol-5-yl | H | 1 | H | H | $CF_3O$ | H | H | 198 - 199 |
| 38 | H, F, H, H, H | 1-Méthylindol-5-yl | H | 1 | H | H | Br | H | H | 209 - 210 |

(suite)

| N° | $X_1$, $X_2$, $X_3$, $X_4$, $X_5$ | R | Y | n | $Z_1$ | $Z_2$ | $Z_3$ | $Z_4$ | $Z_5$ | PF (°C) |
|---|---|---|---|---|---|---|---|---|---|---|
| 39 | H, H, H, H, H | 1-Méthylindol-5-yl | H | 0 | H | Me | Me | H | H | 148 - 150 |
| 40 | H, F, H, H, H | 1-Méthylindol-5-yl | H | 2 | H | H | H | H | H | 158 - 159 |
| 41 | H, $CF_3O$, H, H, H | 1-Méthylindol-5-yl | H | 0 | H | iPr | H | H | H | 164 - 165 |
| 42 | H, $CF_3$, H, H, H | 1-Méthylindol-5-yl | H | 1 | H | $CF_3$ | H | H | H | 197 - 198 |
| 43 | H, F, H, H, H | 1-Méthylindol-5-yl | H | 0 | H | $CF_3$ | H | H | H | 131 - 132 |
| 44 | H, $CF_3$, H, H, H | 1-Méthylindol-5-yl | H | 1 | H | F | H | H | H | 181 - 182 |
| 45 | H, H, H, H, H | 1-Méthylindol-6-yl | H | 0 | H | Me | H | Me | H | 161 - 163 |
| 46 | H, H, H, H, H | 1,2,3-(triméthyl)indol-5-yl | H | 0 | H | Me | H | Me | H | 179 - 181 |
| 47 | H, H, H, H, H | 1-Méthylindol-4-yl | H | 0 | H | Me | H | Me | H | 94 - 106 |
| 48 | H, F, H, H, H | 1-Méthylindol-6-yl | H | 1 | H | F | H | H | H | 173 - 175 |
| 49 | H, F, H, H, H | 1-Méthylindol-7-yl | H | 1 | H | F | H | H | H | 153 - 155 |
| 50 | H, F, H, H, H | 1-Méthylindol-5-yl | H | 2 | F | H | H | H | H | 187 - 188 |
| 51 | H, F, H, H, H | 1-Méthylindol-5-yl | H | 2 | H | H | F | H | H | 198 - 199 |
| 52 | H, F, H, H, H | 1-Méthylindol-5-yl | H | 3 | H | H | H | H | H | 177 - 178 |
| 53 | H, MeO, H, H, H | 1-Méthylindol-5-yl | H | 1 | H | F | H | H | H | 165 - 166 |
| 54 | H, H, H, H, H | 1-Méthylindol-7-yl | H | 0 | H | Me | H | Me | H | 180 - 182 |
| 55 | H, F, H, H, H | 1,2,3-(triméthyl)indol-5-yl | H | 1 | H | F | H | H | H | 183 - 185 |
| 56 | H, F, H, H, H | 1-Méthylindol-4-yl | H | 1 | H | F | H | H | H | 197 - 199 |
| 57 | H, F, H, H, H | 1,2-(diméthyl)indol-5- yl | H | 1 | H | F | H | H | H | 206 - 208 |
| 58 | H, F, H, H, H | 1-Méthylindol-5-yl | H | 2 | H | H | t-Bu | H | H | 182 - 184 |
| 59 | H, H, MeO, H, H | 1-Méthylindol-5-yl | H | 1 | H | F | H | H | H | 202 - 205 |
| 60 | MeO, H, H, H, H | 1-Méthylindol-5-yl | H | 1 | H | F | H | H | H | 177 - 179 |
| 61 | MeO, H, OMe, H, H | 1-Méthylindol-5-yl | H | 1 | H | F | H | H | H | 183 - 185 |
| 62 | H, Cl, H, H, H | 1-Méthylindol-5-yl | H | 1 | H | F | H | H | H | 201 - 202 |
| 63 | H, Me, H, H, H | 1-Méthylindol-5-yl | H | 1 | H | F | H | H | H | $[MH]^+$ : 412 |

(suite)

| N° | $X_1$, $X_2$, $X_3$, $X_4$, $X_5$ | R | Y | n | $Z_1$ | $Z_2$ | $Z_3$ | $Z_4$ | $Z_5$ | PF (°C) |
|---|---|---|---|---|---|---|---|---|---|---|
| 64 | H, $SO_2$Me, H, H, H | 1-Méthylindol-5-yl | H | 1 | H | F | H | H | H | 221 - 223 |
| 65 | H, $NO_2$, H, H, H | 1-Méthylindol-5-yl | H | 1 | H | F | H | H | H | $[MH]^+$ : 443 |
| 66 | H, F, H, H, H | 1-Isopropylindol-5-yl | H | 1 | H | F | H | H | H | 167 - 168 |
| 67 | F, H, H, H, H | 1-Méthylindol-5-yl | H | 1 | H | F | H | H | H | 184 - 185 |
| 68 | H, Pr, H, H, H | 1-Méthylindol-5-yl | H | 1 | H | F | H | H | H | 190 - 191 |
| 69 | H, $CF_3$, H, H, H | 1-Méthylindol-5-yl | H | 1 | H | H | H | H | H | 193 - 194 |
| 70 | H, F, H, H, H | Indol-5-yl | H | 1 | H | F | H | H | H | 158 - 163 |
| 71 | H, $OCF_3$, H, H. H | 1-Méthylindol-5-yl | H | 1 | H | F | H | H | H | 188 - 189 |
| 72 | Me, H, H, H, H | 1-Méthylindol-5-yl | H | 1 | H | F | H | H | H | 204 - 205 |
| 73 | H, tBu, H, H, H | 1-Méthylindol-5-yl | H | 1 | H | F | H | H | H | 209 - 210 |
| 74 | H, $NH_2$, H, H, H | 1-Méthylindol-5-yl | H | 1 | H | F | H | H | H | 189 - 191 |
| 75 | H, H, Me, H, H | 1-Méthylindol-5-yl | H | 1 | H | F | H | H | H | 206 - 208 |
| 76 | H, H, F, H, H | 1-Méthylindol-5-yl | H | 1 | H | F | H | H | H | 230 - 231 |
| 77 | H, OMe, OMe, H, H | 1-Méthylindol-5-yl | H | 1 | H | F | H | H | H | 243 - 246 |
| 78 | H, H, H, H, H | 1-Méthylindol-5-yl | H | 1 | H | F | H | H | H | 185 - 186 |
| 79 | H, F, H, H, H | 1-Méthylindol-5-yl | H | 1 | H | H | H | H | H | 193 - 194 |
| 80 | H, F, H, H, H | 1-Méthylindol-5-yl | H | 2 | H | $CF_3$ | H | H | H | 172 - 173 |
| 81 | H, F, H, H, H | 1-Méthylindol-5-yl | H | 2 | H | F | H | H | H | 178 - 180 |

Les composés de l'invention ont été soumis à des essais pharmacologiques *in vitro* et *in vivo* qui ont mis en évidence leur intérêt comme substances à activités thérapeutiques.

Test d'inhibition du courant induit par la capsaïcine sur les DRG de rat

- Culture primaire de cellules de ganglions de racine dorsale (DRG) de rat :

Les neurones du DRG expriment naturellement le récepteur TRPV1.
Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/ml gentamicine et 50 ng/ml de NGF, puis déposées sur des lamelles de verre recouvertes de laminine (0.25 x 106 cellules par lamelle) qui sont ensuite placées dans des boîtes 12 puits Coming. Les cellules sont incubées à 37°C en atmosphère humidifiée contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

- Electrophysiologie :

Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 ml/min) à l'aide d'un distributeur de solutions acceptant 8 entrées et dont la sortie unique, constituée par un tube de polyéthylène (ouverture 500μm) est placée à moins de 3 mm de la cellule étudiée. La configuration "cellule entière" de la technique de patch-clamp à été utilisée. Les pipettes en verre borosilicaté (résistance 5-10 MOhms) sont approchées de la cellule grâce à un micromanipulateur piézoélectrique 3D (Burleigh, PC1000). Les courants globaux (potentiel de membrane fixé à -60 mV) sont enregistrés avec un amplificateur Axopatch 1D (Axon Instruments, Foster city, Califomie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les composés à tester sont appliqués seuls à la concentration de 10 nM pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.
Les pourcentages d'inhibition de la réponse capsaïcine (300 nM) sont compris entre 20% et 100% pour les composés les plus actifs de l'invention testés à la concentration de 10 nM (voir quelques exemples dans le tableau 2).
Les composés de l'invention sont donc des antagonistes efficaces in vitro des récepteurs de type TRPV1.

**Tableau 2**

| N°composé | % inhibition en patch DRG |
|---|---|
| 1 | 56 |
| 11 | 48 |

Test d'irritation cornéenne souris

Le caractère irritant de la capsaïcine est aisément apprécié au niveau de la cornée puisque cet organe est un des plus innervés par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance.
On peut en conclure que la durée d'action de la capsaïcine à ces doses est inférieure à 2 minutes.
Résumé de la méthodologie :

Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans du Tween 80 à 10% dans le sérum physiologique. Il apparaît, à partir d'études contrôles que dans ces conditions, le solvant n'a aucun effet.
En pratique, le produit à tester est administré par voie orale, et, avec un délai (temps de prétraitement: t) qui dépend des données de pharmacocinétique, l'animal reçoit l'instillation oculaire de 2 μl d'une solution de capsaïcine à 160 μM préparée comme indiqué ci-dessus. Au cours d'une observation de 2 minutes suivant l'instillation, le nombre de frottements de l'oeil instillé par la patte antérieure ipsilatéral est répertorié.

Pour un animal donné, le pourcentage de protection est calculé comme suit :

P= 100 – ((nombre de grattages observés / nombre moyen de grattages du groupe traité par le solvant) x 100)

Ce pourcentage de protection est moyenné pour chaque groupe d'animaux (n = nombre d'animaux testés avec le composé de l'invention).
Les pourcentages de protection évalués, dans ce modèle, pour les composés de l'invention les plus actifs, utilisés à la dose de 60 mg/kg (po), sont compris entre 8% et 100% (voir quelques exemples dans le tableau 3) :

**Tableau 3**

| n°composé | % P - (t) à 60 mg/kg *(po)* - (n = 8) |
|---|---|
| 1 | 26% - (1h) |
| 14 | 60% - (1h) |

Les résultats de ces essais montrent que les composés les plus actifs de l'invention bloquent les effets induits par la stimulation des récepteurs TRPV1.
Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.
Ainsi, selon un autre de ses aspects, l'invention a pour objet des médicaments qui comprennent un composé de formule (I), ou un sel pharmaceutiquement acceptable, ou encore un hydrate ou un solvate dudit composé.
Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou du cerveau, de l'ischémie (de la moelle et/ou du cerveau), de la neurodégénération, des accidents vasculaires hémorragiques (de la moelle et/ou du cerveau), de la douleur post-stroke.
Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.
Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.
On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.
De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.
Les composés de l'invention peuvent également être utilisés pour la préparation d'un médicament destiné à traiter la dépression.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.
Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule (I)

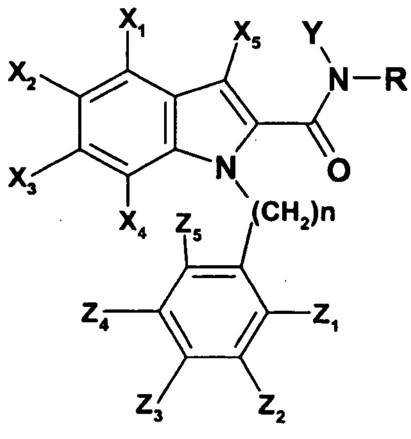

(I)

dans laquelle
$X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_8$-alkyle, $C_3$-$C_7$-cycloalkyle, $C_1$-$C_6$-fluoroalkyle, $C_1$-$C_6$-alcoxy, $C_1$-$C_6$-fluoroalcoxy, cyano, C(O)

$NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle ;
$X_5$ représente un atome d'hydrogène ou d'halogène ou un groupe $C_1$-$C_6$-alkyle, $C_1$-$C_6$-fluoroalkyle ;
R représente un groupe 4-, 5-, 6- ou 7-indolyle,

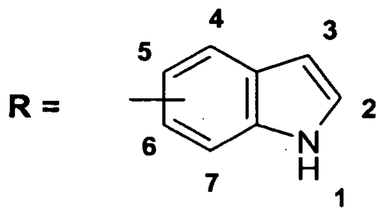

R étant éventuellement substitué en position 1, 2 et/ou 3 par un ou plusieurs groupes choisis parmi les groupes $C_1$-$C_6$-alkyle et $C_1$-$C_6$-fluoroalkyle ;
R étant éventuellement substitué en position 4, 5, 6 et/ou 7 par un ou plusieurs groupes choisis parmi les atomes d'halogène, les groupes $C_1$-$C_6$-alkyle, $C_1$-$C_6$-fluoroalkyle, $C_1$-$C_6$-alcoxy, $C_1$-$C_6$-fluoroalcoxy ;
Y représente un atome d'hydrogène ou un groupe $C_1$-$C_6$-alkyle ;
n est égal à 0, 1, 2 ou 3 ;
$R_1$ et $R_2$ représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkyle, ou aryle ; ou $R_1$ et $R_2$formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkyle ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle ou aryle ;
à l'état de base ou de sel d'addition à un acide, ainsi qu'à l'état d'hydrate ou de solvat.

2. Composé de formule (I) selon la revendication 1, **caractérisé en ce que** $X_1$, $X_2$, $X_3$, $X_4$, $Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_1$-$C_6$-fluoroalkyle, $C_1$-$C_6$-alcoxy, $C_1$-$C_6$-fluoroalcoxy, nitro, $NR_1R_2$, $C_1$-$C_6$-thioalkyle, -S(O)-$C_1$-$C_6$-alkyle, -$S(O)_2$-$C_1$-$C_6$-alkyle ou aryle ;
$X_5$ représente un atome d'hydrogène ou un groupe $C_1$-$C_6$-alkyle ;
R représente un groupe 4-, 5-, 6- ou 7-indolyle,

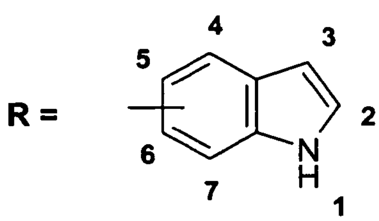

R étant éventuellement substitué en position 1, 2 et/ou 3 par un ou plusieurs groupes $C_1$-$C_6$-alkyle;
Y représente un atome d'hydrogène ;
n est égal à 0, 1, 2 ou 3 ;
$R_1$ et $R_2$ représentent, indépendamment l'un de l'autre, un atome d'hydrogène ;
à l'état de base ou de sel d'addition à un acide, ainsi qu'à l'état d'hydrate ou de solvat.

3. Composé de formule (I) selon la revendication 1 ou 2, **caractérisé en ce que** R représente un groupe indol-5-yle

**R =** 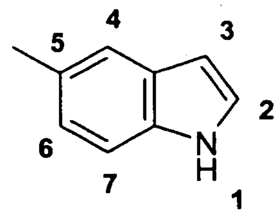

R étant éventuellement substitué en position 1, 2 et/ou 3 par un ou plusieurs groupes choisis parmi les groupes $C_1$-$C_6$-alkyle et $C_1$-$C_6$-fluoroalkyle ;
R étant éventuellement substitué en position 4, 6 et/ou 7 par un ou plusieurs groupes choisis parmi les atomes d'halogène, les groupes $C_1$-$C_6$-alkyle, $C_1$-$C_6$-fluoroalkyle, $C_1$-$C_6$-alcoxy, $C_1$-$C_6$-fluoroalcoxy ;
à l'état de base ou de sel d'addition à un acide, ainsi qu'à l'état d'hydrate ou de solvat.

**4.** Composé de formule (I) selon l'une quelconque des revendications 1 à 3, **caractérisé en ce que** $X_2$ et/ou $X_3$ sont différents d'un atome d'hydrogène ;
à l'état de base ou de sel d'addition à un acide, ainsi qu'à l'état d'hydrate ou de solvat.

**5.** Composé de formule (I) selon l'une quelconque des revendications 1 à 4, **caractérisé en ce que** $X_5$ représente un atome d'hydrogène ;
à l'état de base ou de sel d'addition à un acide, ainsi qu'à l'état d'hydrate ou de solvat.

**6.** Composé de formule (I) selon l'une quelconque des revendications 1 à 5, **caractérisé en ce que** Y représente un atome d'hydrogène ;
à l'état de base ou de sel d'addition à un acide, ainsi qu'à l'état d'hydrate ou de solvat.

**7.** Composé de formule (I) selon la revendication 1 choisi parmi :

*N*-(1-Méthyl-1*H*-indol-5-yl)-1-(3-trifluorométhyl-benzyl)-1*H*-indole-2-carboxamide ;
*N*-(1-Méthyl-1*H*-indol-5-yl)-5-méthoxy-1-(3-trifluorométhyl-benzyl)-1*H*-indole-2-carboxamide ;
*N*-(1-Méthyl-1*H*-indol-5-yl)-5-fluoro-1-(3-fluoro-benzyl)-1*H*-indole-2-carboxamide ;
*N*-(1-Méthyl-1*H*-indol-5-yl)-1-(3-trifluorométhyl-phényl)-1*H*-indole-2-carboxamide ;
*N*-(1-Méthyl-1H-indol-5-yl)-1-(3,5-diméthyl-phényl)-1*H*-indole-2-carboxamide ;
*N*-(1-Méthyl-1*H*-indol-5-yl)-5-méthyl-1-(3-trifluorométhyl-benzyl)-1*H*-indole-2-carboxamide;
*N*-(1-Méthyl-1*H*-indol-5-yl)-1-phényl-1*H*-indole-2-carboxamide ;
*N*-(1-Méthyl-1*H*-indol-5-yl)-6-méthoxy-1-(3-trifluorométhyl-benzyl)-1*H*-indole-2-carboxamide ;
*N*-(1-Méthyl-1*H*-indol-5-yl)-5-chloro-1-(3-trifluorométhyl-benzyl)-1*H*-indole-2-carboxamide;
*N*-(1-Méthyl-1*H*-indol-5-yl)-4-méthoxy-1-(3-trifluorométhyl-benzyl)-1*H*-indole-2-carboxamide ;
*N*-(1-Méthyl-1*H*-indol-5-yl)-5-fluoro-1-(3-trifluorométhyl-benzyl)-1*H*-indole-2-carboxamide ;
*N*-(1-Méthyl-1*H*-indol-5-yl)-5-fluoro-1-(4-trifluorométhyl-benzyl)-1*H*-indole-2-carboxamide ;
*N*-(1-Méthyl-1*H*-indol-5-yl)-1-(6-chloro-3-trifluorométhyl-benzyl)-5-fluoro-1*H*-indole-2-carboxamide ;
*N*-(1-Méthyl-1*H*-indol-5-yl)-5-fluoro-1-(3-méthyl-benzyl)-1*H*-indole-2-carboxamide ;
*N*-(1-Méthyl-1*H*-indol-5-yl)-5-fluoro-1-(3-méthoxy-benzyl)-1*H*-indole-2-carboxamide ;
*N*-(1-Méthyl-1*H*-indol-5-yl)-5-fluoro-1-(3-trifluorométhoxy-benzyl)-1*H*-indole-2-carboxamide ;
*N*-(1-Méthyl-1*H*-indol-5-yl)-1-(4-*tert*-butyl-benzyl)-5-fluoro-1*H*-indole-2-carboxamide ;
*N*-(1-Méthyl-1*H*-indol-5-yl)-1-(3-chloro-benzyl)-5-fluoro-1*H*-indole-2-carboxamide ;
*N*-(1-Méthyl-1*H*-indol-5-yl)-1-(4-fluoro-phényl)-1*H*-indole-2-carboxamide ;
*N*-(1-Méthyl-1*H*-indol-5-yl)-1-(4-méthylthio-phényl)-1*H*-indole-2-carboxamide ;
*N*-(1-Méthyl-1*H*-indol-5-yl)-1-(4-sec-butyl-phényl)-1*H*-indole-2-carboxamide ;
*N*-(1-Méthyl-1*H*-indol-5-yl)-5-méthoxy-1-phényl-1*H*-indole-2-carboxamide ;
*N*-(1-Méthyl-1*H*-indol-5-yl)-1-(4-isopropyl-phényl)-6-méthyl-1*H*-indole-2-carboxamide ;
*N*-(1-Méthyl-1*H*-indol-5-yl)-1-(4-n-pentyl-phényl)-1*H*-indole-2-carboxamide ;
*N*-(1-Méthyl-1*H*-indol-5-yl)-1-(4-cyclopentyl-phényl)-1*H*-indole-2-carboxamide ;
*N*-(1-Méthyl-1*H*-indol-5-yl)-1-(biphényl-4-yl)-1*H*-indole-2-carboxamide ;
*N*-(1-Méthyl-1*H*-indol-5-yl)-1-(4-trifluorométhyl-phényl)-1*H*-indole-2-carboxamide ;
*N*-(1-Méthyl-1*H*-indol-5-yl)-1-(4-n-propyl-phényl)-1*H*-indole-2-carboxamide
*N*-(1-Méthyl-1*H*-indol-5-yl)-1-(3-méthyl-phényl)-1*H*-indole-2-carboxamide ;

*N*-(1-Méthyl-1*H*-indol-5-yl)-1-(4-isopropyl-phényl)-1*H*-indole-2-carboxamide ;
*N*-(1-Méthyl-1*H*-indol-5-yl)-1-(4-*tert*-butyl-phényl)-1*H*-indole-2-carboxamide ;
*N*-(1-Méthyl-1*H*-indol-5-yl)-1-(4-cyclohexyl-phényl)-1*H*-indole-2-carboxamide ;
*N*-(1-Méthyl-1*H*-indol-5-yl)-1-(4-éthoxy-phényl)-1*H*-indole-2-carboxamide ;
*N*-(1-Méthyl-1*H*-indol-5-yl)-1-(4-chloro-phényl)-1*H*-indole-2-carboxamide ;
*N*-(1-Méthyl-1*H*-indol-5-yl)-1-(3,5-difluoro-benzyl)-5-fluoro-1*H*-indole-2-carboxamide ;
*N*-(1-Méthyl-1*H*-indol-5-yl)-1-(2-fluoro-benzyl)-5-fluoro-1*H*-indole-2-carboxamide ;
*N*-(1-Méthyl-1*H*-indol-5-yl)-1-(4-trifluorométhoxy-benzyl)-5-fluoro-1*H*-indole-2-carboxamide ;
*N*-(1-Méthyl-1*H*-indol-5-yl)-1-(4-bromo-benzyl)-5-fluoro-1*H*-indole-2-carboxamide
*N*-(1-Méthyl-1*H*-indol-5-yl)-1-(3,4-diméthyl-phényl)-1*H*-indole-2-carboxamide ;
*N*-(1-Méthyl-1*H*-indol-5-yl)-1-(phénéthyl)-5-fluoro-1*H*-indole-2-carboxamide ;
*N*-(1-Méthyl-1*H*-indol-5-yl)-1-(3-isopropyl-phényl)-5-trifluorométhoxy-1*H*-indole-2-carboxamide ;
*N*-(1-Méthyl-1*H*-indol-5-yl)-1-(3-trifluorométhyl-benzyl)-5-trifluorométhyl-1*H*-indole-2-carboxamide ;
*N*-(1-Méthyl-1*H*-indol-5-yl)-1-(3-trifluorométhyl-phényl)-5-fluoro-1*H*-indole-2-carboxamide;
*N*-(1-Méthyl-1*H*-indol-5-yl)-1-(3-fluoro-benzyl)-5-trifluorométhyl-1*H*-indole-2-carboxamide ;
*N*-(1-Méthyl-1*H*-indol-6-yl)-1-(3,5-diméthyl-phényl)-1*H*-indole-2-carboxamide ;
*N*-(1,2,3-Triméthyl-1H-indol-5-yl)-1-(3,5-diméthyl-phényl)-1*H*-indole-2-carboxamide ;
*N*-(1-Méthyl-1*H*-indol-4-yl)-1-(3,5-diméthyl-phényl)-1*H*-indole-2-carboxamide ;
*N*-(1-Méthyl-1*H*-indol-6-yl)-1-(3-fluoro-benzyl)-5-fluoro-1*H*-indole-2-carboxamide ;
*N*-(1-Méthyl-1*H*-indol-7-yl)-1-(3-fluoro-benzyl)-5-fluoro-1*H*-indole-2-carboxamide ;
*N*-(1-Méthyl-1*H*-indol-5-yl)-1-[2-(2-fluoro-phényl)éthyl]-5-fluoro-1*H*-indole-2-carboxamide ;
*N*-(1-Méthyl-1*H*-indol-5-yl)-1-[2-(4-fluoro-phényl)éthyl]-5-fluoro-1*H*-indole-2-carboxamide ;
*N*-(1-Méthyl-1*H*-indol-5-yl)-1-(3-phénylpropyl)-5-fluoro-1*H*-indole-2-carboxamide ;
*N*-(1-Méthyl-1*H*-indol-5-yl)-1-(3-fluoro-benzyl)-5-méthoxy-1*H*-indole-2-carboxamide ;
*N*-(1-Méthyl-1*H*-indol-7-yl)-1-(3,5-diméthyl-phényl)-1*H*-indole-2-carboxamide ;
*N*-(1,2,3-Triméthyl-1*H*-indol-5-yl)-1-(3-fluoro-benzyl)-5-fluoro-1*H*-indole-2-carboxamide
*N*-(1-Méthyl-1*H*-indol-4-yl)-1-(3-fluoro-benzyl)-5-fluoro-1*H*-indole-2-carboxamide ;
*N*-(1,2-Diméthyl-1*H*-indol-5-yl)-1-(3-fluoro-benzyl)-5-fluoro-1*H* -indole-2-carboxamide ;
*N*-(1-Méthyl-1*H*-indol-5-yl)-1-[2-(4-*tert*-butyliphényl)éthyl]-5-fluoro-1*H*-indole-2-carboxamide ;
*N*-(1-Méthyl-1*H*-indol-5-yl)-1-(3-fluoro-benzyl)-6-méthoxy-1*H*-indole-2-carboxamide ;
*N*-(1-Méthyl-1*H*-indol-5-yl)-1-(3-fluoro-benzyl)-4-méthoxy-1*H*-indole-2-carboxamide ;
*N*-(1-Méthyl-1*H*-indol-5-yl)-1-(3-fluoro-benzyl)-4,6-diméthoxy-1*H*-indole-2-carboxamide ;
*N*-(1-Méthyl-1*H*-indol-5-yl)-1-(3-fluoro-benzyl)-5-chloro-1*H*-indole-2-carboxamide ;
*N*-(1-Méthyl-1*H*-indol-5-yl)-1-(3-fluoro-benzyl)-5-méthyl-1*H*-indole-2-carboxamide ;
*N*-(1-Méthyl-1*H*-indol-5-yl)-1-(3-fluoro-benzyl)-5-méthylsulfonyl-1*H*-indole-2-carboxamide;
*N*-(1-Méthyl-1*H*-indol-5-yl)-1-(3-fluoro-benzyl)-5-nitro-1*H*-indole-2-carboxamide ;
*N*-(1-Isopropyl-1*H*-indol-5-yl)-1-(3-fluoro-benzyl)-5-fluoro-1*H*-indole-2-carboxamide ;
*N*-(1-Méthyl-1*H*-indol-5-yl)-1-(3-fluoro-benzyl)-4-fluoro-1*H*-indole-2-carboxamide ;
*N*-(1-Méthyl-1*H*-indol-5-yl)-1-(3-fluoro-benzyl)-5-isopropyl-1*H*-indole-2-carboxamide ;
*N*-(1-Méthyl-1*H*-indol-5-yl)-1-benzyl-5-trifluorométhyl-1*H*-indole-2-carboxamide ;
*N*-(1*H*-Indol-5-yl)-5-fluoro-1-(3-fluoro-benzyl)-1*H*-indole-2-carboxamide ;
*N*-(1-Méthyl-1*H*-indol-5-yl)-1-(3-fluoro-benzyl)-5-trifluorométhoxy-1*H*-indole-2-carboxamide ;
*N*-(1-Méthyl-1*H*-indol-5-yl)-1-(3-fluoro-benzyl)-4-méthyl-1*H*-indole-2-carboxamide ;
*N*-(1-Méthyl-1*H*-indol-5-yl)-1-(3-fluoro-benzyl)-5-*tert*-butyl-1*H*-indole-2-carboxamide ;
*N*-(1-Méthyl-1*H*-indol-5-yl)-1-(3-fluoro-benzyl)-5-amino-1*H*-indole-2-carboxamide ;
*N*-(1-Méthyl-1*H*-indol-5-yl)-1-(3-fluoro-benzyl)-6-méthyl-1*H*-indole-2-carboxamide ;
*N*-(1-Méthyl-1*H*-indol-5-yl)-1-(3-fluoro-benzyl)-6-fluoro-1*H*-indole-2-carboxamide ;
*N*-(1-Méthyl-1*H*-indol-5-yl)-1-(3-fluoro-benzyl)-5,6-diméthoxy-1*H*-indole-2-carboxamide ;
*N*-(1-Méthyl-1*H*-indol-5-yl)-1-(3-fluoro-benzyl)-1*H*-indole-2-carboxamide ;
*N*-(1-Méthyl-1*H*-indol-5-yl)-1-benzyl-5-fluoro-1*H*-indole-2-carboxamide ;
*N*-(1-Méthyl-1*H*-indol-5-yl)-1-[2-(3-trifluorométhyl-phényl)éthyl]-5-fluoro-1*H*-indole-2-carboxamide ;
*N*-(1-Méthyl-1*H*-indol-5-yl)-1-[2-(3-fluoro-phényl)éthyl]-5-fluoro-1*H*-indole-2-carboxamide.

**8.** Procédé de préparation d'un composé de formule (I) selon la revendication 1, **caractérisé en ce que** l'on fait réagir un composé de formule générale (IV)

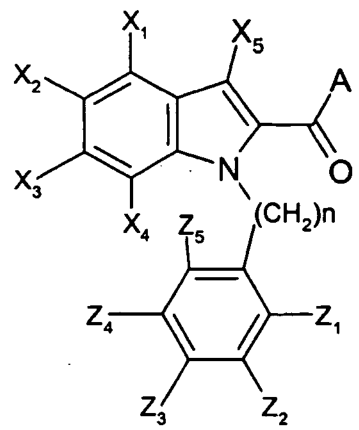

(IV)

dans laquelle $X_1$, $X_2$, $X_3$, $X_4$, $X_5$, $Z_1$, $Z_2$, $Z_3$, $Z_4$, $Z_5$ et n sont tels que définis dans la formule générale (I) selon la revendication 1 et A représente un groupe $C_1$-$C_4$-alcoxy,
avec un amidure du composé de formule générale (V)

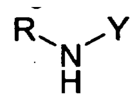

(V)

dans laquelle R et Y sont tels que définis dans la formule générale (I) selon la revendication 1,
au reflux d'un solvant, l'amidure du composé de formule générale (V) étant préparé par action préalable du triméthylaluminium sur les aminoindoles de formule générale (V).

**9.** Procédé de préparation d'un composé de formule (I) selon la revendication 1, **caractérisé en ce que** l'on transforme un composé de formule générale (IV)

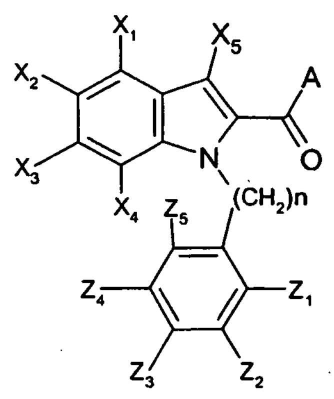

(IV)

dans laquelle $X_1$, $X_2$, $X_3$, $X_4$, $X_5$, $Z_1$, $Z_2$, $Z_3$, $Z_4$, $Z_5$ et n sont tels que définis dans la formule générale (I) selon la revendication 1 et A représente un groupe hydroxy,
en chlorure d'acide par action du chlorure de thionyle au reflux d'un solvant,
puis **en ce que** l'on fait réagir, en présence d'une base, le composé de formule générale (IV) obtenu, dans laquelle $X_1$, $X_2$, $X_3$, $X_4$, $X_5$, $Z_1$, $Z_2$, $Z_3$, $Z_4$, $Z_5$ et n sont tels que définis dans la formule générale (I) selon la revendication 1 et A représente un atome de chlore, avec l'aminoindole de formule générale (V),

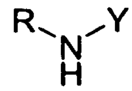

**(V)**

dans laquelle R et Y sont tels que définis dans la formule générale (I) selon la revendication 1, ou bien **en ce que** l'on effectue une réaction de couplage entre un composé de formule générale (IV) dans laquelle $X_1$, $X_2$, $X_3$, $X_4$, $X_5$, $Z_1$, $Z_2$, $Z_3$, $2_4$, $Z_5$ et n sont tels que définis dans la formule générale (I) selon la revendication 1 et A représente un groupe hydroxy,
et l'aminoindole de formule générale (V), dans laquelle R et Y sont tels que définis dans la formule générale (I) selon la revendication 1, en présence d'un agent de couplage et d'une base, dans un solvant.

**10.** Médicament, **caractérisé en ce qu'**il comprend un composé de formule (I), selon l'une quelconque des revendications 1 à 7, ou un sel pharmaceutiquement acceptable, ou encore un hydrate ou un solvat du composé de formule (I).

**11.** Composition pharmaceutique, **caractérisée en ce qu'**elle comprend un composé de formule (I), selon l'une quelconque des revendications 1 à 7, ou un sel pharmaceutiquement acceptable, un hydrate ou un solvat de ce composé, ainsi qu'au moins un excipient pharmaceutiquement acceptable.

**12.** Utilisation d'un composé de formule (I), selon l'une quelconque des revendications 1 à 7, pour la préparation d'un médicament destiné à prévenir ou à traiter les pathologies dans lesquelles les récepteurs de type TRPV1 sont impliqués.

**13.** Utilisation d'un composé de formule (I), selon la revendication 12, pour la préparation d'un médicament destiné à prévenir ou à traiter la douleur et l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona, ou à traiter la dépression.

**14.** Utilisation d'un composé de formule (I), selon la revendication 13, **caractérisé en ce que** la douleur est de nature chronique, neuropathique (traumatique, diabétique, métabolique, infectieuse, toxique, induite par un traitement anticancéreux ou hiatrogène), (ostéo-) arthritique, rhumatismale, ou bien de type fibromyalgies, douleur du dos, douleur liée au cancer, névralgie faciale, céphalées, migraine, douleur dentaire, brûlure, coup de soleil, morsure ou piqûre, nevralgie post-herpétique, douleur musculaire, compression nerveuse (centrale et/ou périphérique), traumatismes de la moelle et/ou du cerveau, l'ischémie (de la moelle et/ou du cerveau), neurodégénération, accidents vasculaires hémorragiques (de la moelle et/ou du cerveau), douleur post-stroke.

**15.** Utilisation d'un composé de formule (I), selon la revendication 13, **caractérisé en ce que** les désordres urologiques sont choisis parmi l'hyperactivité de la vessie, l'hyperéflexie vésicale, l'instabilité vésicale, l'incontinence, la miction d'urgence, l'incontinence urinaire, la cystite, la colique néphrétique, l'hypersensibilité pelvienne et la douleur pelvienne.

**16.** Utilisation d'un composé de formule (I), selon la revendication 13, **caractérisé en ce que** les désordres gynécologiques sont choisis parmi la vulvodynie, les douleurs liées aux salpingites, aux dysménorrhées.

**17.** Utilisation d'un composé de formule (I), selon la revendication 13, **caractérisé en ce que** les désordres gastrointestinaux sont choisis parmi le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

**18.** Utilisation d'un composé de formule (I), selon la revendication 13, **caractérisé en ce que** les désordres respiratoires sont choisis parmi l'asthme, la toux, la COPD, la bronchoconstriction et les désordres inflammatoires.

## Claims

**1.** Compound corresponding to the formula (I)

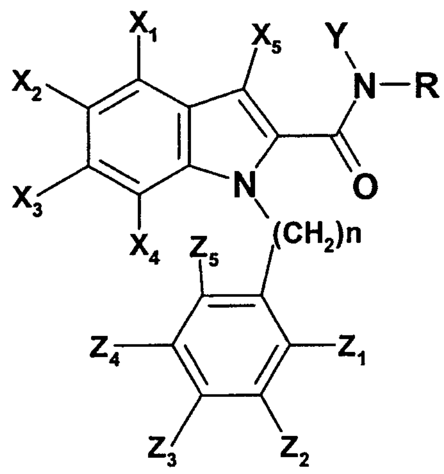

(I)

in which

$X_1$, $X_2$, $X_3$, $X_4$, $Z_1$, $Z_2$, $Z_3$, $Z_4$ and $Z_5$ represent, independently of one another, a hydrogen or halogen atom or a $C_1$-$C_6$ alkyl, $C_3$-$C_7$ cycloalkyl, $C_1$-$C_6$ fluoroalkyl, $C_1$-$C_6$ alkoxy, $C_1$-$C_6$ fluoroalkoxy, cyano, $C(O)NR_1R_2$, nitro, $NR_1R_2$, $C_1$-$C_6$ thioalkyl, -S(O)-($C_1$-$C_6$)alkyl, -$S(O)_2$-($C_1$-$C_6$)alkyl, $SO_2NR_1R_2$, $NR_3COR_4$, $NR_3SO_2R_5$ or aryl group;

$X_5$ represents a hydrogen or halogen atom or a $C_1$-$C_6$ alkyl or $C_1$-$C_6$ fluoroalkyl group;

R represents a 4-, 5-, 6- or 7-indolyl group,

R = 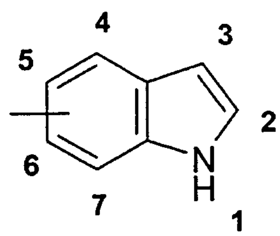

R optionally being substituted in the 1, 2 and/or 3 position by one or more groups chosen from the $C_1$-$C_6$ alkyl and $C_1$-$C_6$ fluoroalkyl groups;

R optionally being substituted in the 4, 5, 6 and/or 7 position by one or more groups chosen from halogen atoms or $C_1$-$C_6$ alkyl, $C_1$-$C_6$ fluoroalkyl, $C_1$-$C_6$ alkoxy or $C_1$-$C_6$ fluoroalkoxy groups;

Y represents a hydrogen atom or a $C_1$-$C_6$ alkyl group;

n is equal to 0, 1, 2 or 3;

$R_1$ and $R_2$ represent, independently of one another, a hydrogen atom or a $C_1$-$C_6$ alkyl, $C_3$-$C_7$ cycloalkyl, ($C_3$-$C_7$)cycloalkyl($C_1$-$C_3$)alkyl or aryl group; or $R_1$ and $R_2$ form, together with the nitrogen atom which carries them, an azetidine, pyrrolidine, piperidine, azepine, morpholine, thiomorpholine, piperazine or homopiperazine group, this group optionally being substituted by a $C_1$-$C_6$ alkyl, $C_3$-$C_7$ cycloalkyl, ($C_3$-$C_7$)cycloalkyl($C_1$-$C_3$)alkyl or aryl group;

$R_3$ and $R_4$ represent, independently of one another, a hydrogen atom or a $C_1$-$C_6$ alkyl or aryl group;

$R_5$ represents a $C_1$-$C_6$ alkyl or aryl group;

in the form of the base or of an addition salt with an acid, and in the hydrate or solvate form.

**2.** Compound of formula (I) according to Claim 1, **characterized in that**

$X_1$, $X_2$, $X_3$, $X_4$, $Z_1$, $Z_2$, $Z_3$, $Z_4$ and $Z_5$ represent, independently of one another, a hydrogen or halogen atom or a $C_1$-$C_6$ alkyl, $C_3$-$C_7$ cycloalkyl, $C_1$-$C_6$ fluoroalkyl, $C_1$-$C_6$ alkoxy, $C_1$-$C_6$ fluoroalkoxy, nitro, $NR_1R_2$, $C_1$-$C_6$ thioalkyl, -S (O)-($C_1$-$C_6$)alkyl, -$S(O)_2$-($C_1$-$C_6$)alkyl or aryl group;

$X_5$ represents a hydrogen atom or a $C_1$-$C_6$ alkyl group;

R represents a 4-, 5-, 6- or 7-indolyl group,

R = 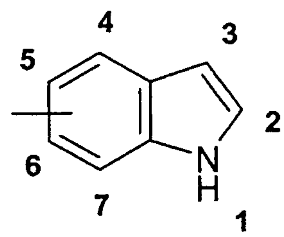

R optionally being substituted in the 1, 2 and/or 3 position by one or more $C_1$-$C_6$ alkyl groups;
Y represents a hydrogen atom;
n is equal to 0, 1, 2 or 3;
$R_1$ and $R_2$ represent, independently of one another, a hydrogen atom;
in the form of the base or of an addition salt with an acid, and in the hydrate or solvate form.

**3.** Compound of formula (I) according to Claim 1 or 2, **characterized in that** R represents an indol-5-yl group

R = 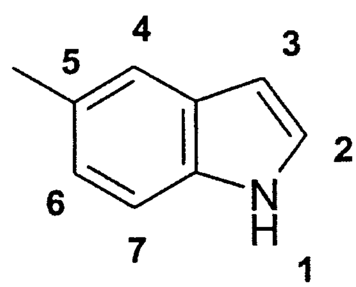

R optionally being substituted in the 1, 2 and/or 3 position by one or more groups chosen from $C_1$-$C_6$ alkyl and $C_1$-$C_6$ fluoroalkyl groups;
R optionally being substituted in the 4, 6 and/or 7 position by one or more groups chosen from halogen atoms or $C_1$-$C_6$ alkyl, $C_1$-$C_6$ fluoroalkyl, $C_1$-$C_6$ alkoxy or $C_1$-$C_6$ fluoroalkoxy groups;
in the form of the base or of an addition salt with an acid, and in the hydrate or solvate form.

**4.** Compound of formula (I) according to any one of Claims 1 to 3, **characterized in that** $X_2$ and/or $X_3$ are other than a hydrogen atom;
in the form of the base or of an addition salt with an acid, and in the hydrate or solvate form.

**5.** Compound of formula (I) according to any one of Claims 1 to 4, **characterized in that** $X_5$ represents a hydrogen atom;
in the form of the base or of an addition salt with an acid, and in the hydrate or solvate form.

**6.** Compound of formula (I) according to any one of Claims 1 to 5, **characterized in that** Y represents a hydrogen atom;
in the form of the base or of an addition salt with an acid, and in the hydrate or solvate form.

**7.** Compound of formula (I) according to Claim 1, chosen from:

*N*-(1-Methyl-1*H*-indol-5-yl)-1-[3-(trifluoromethyl)-benzyl]-1*H*-indole-2-carboxamide;
*N*-(1-Methyl-1*H*-indol-5-yl)-5-methoxy-1-[3-(trifluoromethyl)benzyl]-1*H*-indole-2-carboxamide;
*N*-(1-Methyl-1*H*-indol-5-yl)-5-fluoro-1-(3-fluorobenzyl)-1H-indole-2-carboxamide;
*N*-(1-Methyl-1*H*-indol-5-yl)-1-[3-(trifluoromethyl)-phenyl]-1*H*-indole-2-carboxamide;
*N*-(1-Methyl-1*H*-indol-5-yl)-1-(3,5-dimethylphenyl)-1*H*-indole-2-carboxamide;
*N*-(1-Methyl-1*H*-indol-5-yl)-5-methyl-1-[3-(trifluoromethyl)benzyl]-1*H*-indole-2-carboxamide;
*N*-(1-Methyl-1*H*-indol-5-yl)-1-phenyl-1*H*-indole-2-carboxamide;
*N*-(1-Methyl-1*H*-indol-5-yl)-6-methoxy-1-[3-(trifluoromethyl)benzyl]-1*H*-indole-2-carboxamide;
*N*-(1-Methyl-1*H*-indol-5-yl)-5-chloro-1-[3-(trifluoromethyl)benzyl]-1*H*-indole-2-carboxamide;
*N*-(1-Methyl-1*H*-indol-5-yl)-4-methoxy-1-[3-(trifluoromethyl)benzyl]-1*H*-indole-2-carboxamide;

*N*-(1-Methyl-1*H*-indol-5-yl)-5-fluoro-1-[3-(trifluoromethyl)benzyl]-1*H*-indole-2-carboxamide;
*N*-(1-Methyl-1*H*-indol-5-yl)-5-fluoro-1-[4-(trifluoromethyl)benzyl]-1*H*-indole-2-carboxamide;
*N*-(1-Methyl-1*H*-indol-5-yl)-1-[6-chloro-3-(trifluoromethyl)benzyl]-5-fluoro-1*H*-indole-2-carboxamide;
*N*-(1-Methyl-1*H*-indol-5-yl)-5-fluoro-1-(3-methylbenzyl)-1*H*-indole-2-carboxamide;
*N*-(1-Methyl-1*H*-indol-5-yl)-5-fluoro-1-(3-methoxybenzyl)-1*H*-indole-2-carboxamide;
*N*-(1-Methyl-1*H*-indol-5-yl)-5-fluoro-1-[3-(trifluoromethoxy)benzyl]-1*H*-indole-2-carboxamide;
*N*-(1-Methyl-1*H*-indol-5-yl)-1-[4-(tert-butyl)benzyl]-5-fluoro-1*H*-indole-2-carboxamide;
*N*-(1-Methyl-1*H*-indol-5-yl)-1-(3-chlorobenzyl)-5-fluoro-1*H*-indole-2-carboxamide;
*N*-(1-Methyl-1*H*-indol-5-yl)-1-(4-fluorophenyl)-1*H*-indole-2-carboxamide;
*N*-(1-Methyl-1*H*-indol-5-yl)-1-[4-(methylthio)phenyl]-1*H*-indole-2-carboxamide;
*N*-(1-Methyl-1*H*-indol-5-yl)-1-[4-(sec-butyl)phenyl]-1*H*-indole-2-carboxamide;
*N*-(1-Methyl-1*H*-indol-5-yl)-5-methoxy-1-phenyl-1*H*-indole-2-carboxamide;
*N*-(1-Methyl-1*H*-indol-5-yl)-1-(4-isopropylphenyl)-6-methyl-1*H*-indole-2-carboxamide;
*N*-(1-Methyl-1*H*-indol-5-yl)-1-[4-(n-pentyl)phenyl]-1*H*-indole-2-carboxamide;
*N*-(1-Methyl-1*H*-indol-5-yl)-1-(4-cyclopentylphenyl)-1*H*-indole-2-carboxamide;
*N*-(1-Methyl-1*H*-indol-5-yl)-1-(biphenyl-4-yl)-1*H*-indole-2-carboxamide;
*N*-(1-Methyl-1*H*-indol-5-yl)-1-[4-(trifluoromethyl)-phenyl]-1*H*-indole-2-carboxamide;
*N*-(1-Methyl-1*H*-indol-5-yl)-1-[4-(n-propyl)phenyl]-1*H*-indole-2-carboxamide;
*N*-(1-Methyl-1*H*-indol-5-yl)-1-(3-methylphenyl)-1*H*-indole-2-carboxamide;
*N*-(1-Methyl-1*H*-indol-5-yl)-1-(4-isopropylphenyl)-1*H*-indole-2-carboxamide;
*N*-(1-Methyl-1*H*-indol-5-yl)-1-[4-(tert-butyl)phenyl]-1*H*-indole-2-carboxamide;
*N*-(1-Methyl-1*H*-indol-5-yl)-1-(4-cyclohexylphenyl)-1*H*-indole-2-carboxamide;
*N*-(1-Methyl-1*H*-indol-5-yl)-1-(4-ethoxyphenyl)-1*H*-indole-2-carboxamide;
*N*-(1-Methyl-1*H*-indol-5-yl)-1-(4-chlorophenyl)-1*H*-indole-2-carboxamide;
*N*-(1-Methyl-1*H*-indol-5-yl)-1-(3,5-difluorobenzyl)-5-fluoro-1*H*-indole-2-carboxamide;
*N*-(1-Methyl-1*H*-indol-5-yl)-1-(2-fluorobenzyl)-5-fluoro-1*H*-indole-2-carboxamide;
*N*-(1-Methyl-1*H*-indol-5-yl)-1-[4-(trifluoromethoxy)-benzyl]-5-fluoro-1*H*-indole-2-carboxamide;
*N*-(1-Methyl-1*H*-indol-5-yl)-1-(4-bromobenzyl)-5-fluoro-1*H*-indole-2-carboxamide;
*N*-(1-Methyl-1*H*-indol-5-yl)-1-(3,4-dimethylphenyl)-1*H*-indole-2-carboxamide;
*N*-(1-Methyl-1*H*-indol-5-yl)-1-phenylethyl-5-fluoro-1*H*-indole-2-carboxamide;
*N*-(1-Methyl-1*H*-indol-5-yl)-1-(3-isopropylphenyl)-5-trifluoromethoxy-1*H*-indole-2-carboxamide;
*N*-(1-Methyl-1*H*-indol-5-yl)-1-[3-(trifluoromethyl)-benzyl]-5-trifluoromethyl-1*H*-indole-2-carboxamide;
*N*-(1-Methyl-1*H*-indol-5-yl)-1-[3-(trifluoromethyl)-phenyl]-5-fluoro-1*H*-indole-2-carboxamide;
*N*-(1-Methyl-1*H*-indol-5-yl)-1-(3-fluorobenzyl)-5-trifluoromethyl-1*H*-indole-2-carboxamide;
*N*-(1-Methyl-1*H*-indol-6-yl)-1-(3,5-dimethylphenyl)-1*H*-indole-2-carboxamide;
*N*-(1,2,3-Trimethyl-1*H*-indol-5-yl)-1-(3,5-dimethylphenyl)-1*H*-indole-2-carboxamide;
*N*-(1-Methyl-1*H*-indol-4-yl)-1-(3,5-dimethylphenyl)-1*H*-indole-2-carboxamide;
*N*-(1-Methyl-1*H*-indol-6-yl)-1-(3-fluorobenzyl)-5-fluoro-1*H*-indole-2-carboxamide;
*N*-(1-Methyl-1*H*-indol-7-yl)-1-(3-fluorobenzyl)-5-fluoro-1*H*-indole-2-carboxamide;
*N*-(1-Methyl-1*H*-indol-5-yl)-1-[2-(2-fluorophenyl)ethyl]-5-fluoro-1*H*-indole-2-carboxamide;
*N*-(1-Methyl-1*H*-indol-5-yl)-1-[2-(4-fluorophenyl)ethyl]-5-fluoro-1*H*-indole-2-carboxamide;
*N*-(1-Methyl-1*H*-indol-5-yl)-1-(3-phenylpropyl)-5-fluoro-1*H*-indole-2-carboxamide;
*N*-(1-Methyl-1*H*-indol-5-yl)-1-(3-fluorobenzyl)-5-methoxy-1*H*-indole-2-carboxamide;
*N*-(1-Methyl-1*H*-indol-7-yl)-1-(3,5-dimethylphenyl)-1*H*-indole-2-carboxamide;
*N*-(1,2,3-Trimethyl-1*H*-indol-5-yl)-1-(3-fluorobenzyl)-5-fluoro-1*H*-indole-2-carboxamide;
*N*-(1-Methyl-1*H*-indol-4-yl)-1-(3-fluorobenzyl)-5-fluoro-1*H*-indole-2-carboxamide;
*N*-(1,2-Dimethyl-1*H*-indol-5-yl)-1-(3-fluorobenzyl)-5-fluoro-1*H*-indole-2-carboxamide;
*N*-(1-Methyl-1*H*-indol-5-yl)-1-{2-[4-(tert-butyl)phenyl]-ethyl}-5-fluoro-1*H*-indole-2-carboxamide;
*N*-(1-Methyl-1*H*-indol-5-yl)-1-(3-fluorobenzyl)-6-methoxy-1*H*-indole-2-carboxamide;
*N*-(1-Methyl-1*H*-indol-5-yl)-1-(3-fluorobenzyl)-4-methoxy-1*H*-indole-2-carboxamide;
*N*-(1-Methyl-1*H*-indol-5-yl)-1-(3-fluorobenzyl)-4,6-dimethoxy-1*H*-indole-2-carboxamide;
*N*-(1-Methyl-1*H*-indol-5-yl)-1-(3-fluorobenzyl)-5-chloro-1*H*-indole-2-carboxamide;
*N*-(1-Methyl-1*H*-indol-5-yl)-1-(3-fluorobenzyl)-5-methyl-1*H*-indole-2-carboxamide;
*N*-(1-Methyl-1*H*-indol-5-yl)-1-(3-fluorobenzyl)-5-methylsulphonyl-1*H*-indole-2-carboxamide;
*N*-(1-Methyl-1*H*-indol-5-yl)-1-(3-fluorobenzyl)-5-nitro-1*H*-indole-2-carboxamide;
*N*-(1-Isopropyl-1*H*-indol-5-yl)-1-(3-fluorobenzyl)-5-fluoro-1*H*-indole-2-carboxamide;
*N*-(1-Methyl-1*H*-indol-5-yl)-1-(3-fluorobenzyl)-4-fluoro-1*H*-indole-2-carboxamide;
*N*-(1-Methyl-1*H*-indol-5-yl)-1-(3-fluorobenzyl)-5-isopropyl-1*H*-indole-2-carboxamide;

*N*-(1-Methyl-1*H*-indol-5-yl)-1-benzyl-5-trifluoromethyl-1*H*-indole-2-carboxamide;
*N*-(1*H*-Indol-5-yl)-5-fluoro-1-(3-fluorobenzyl)-1*H*-indole-2-carboxamide;
*N*-(1-Methyl-1*H*-indol-5-yl)-1-(3-fluorobenzyl)-5-trifluoromethoxy-1*H*-indole-2-carboxamide;
*N*-(1-Methyl-1*H*-indol-5-yl)-1-(3-fluorobenzyl)-4-methyl-1*H*-indole-2-carboxamide;
*N*-(1-Methyl-1*H*-indol-5-yl)-1-(3-fluorobenzyl)-5-(tert-butyl)-1*H*-indole-2-carboxamide;
*N*-(1-Methyl-1*H*-indol-5-yl)-1-(3-fluorobenzyl)-5-amino-1H-indole-2-carboxamide;
*N*-(1-Methyl-1*H*-indol-5-yl)-1-(3-fluorobenzyl)-6-methyl-1H-indole-2-carboxamide;
*N*-(1-Methyl-1*H*-indol-5-yl)-1-(3-fluorobenzyl)-6-fluoro-1*H*-indole-2-carboxamide;
*N*-(1-Methyl-1*H*-indol-5-yl)-1-(3-fluorobenzyl)-5,6-dimethoxy-1*H*-indole-2-carboxamide;
*N*-(1-Methyl-1*H*-indol-5-yl)-1-(3-fluorobenzyl)-1*H*-indole-2-carboxamide;
*N*-(1-Methyl-1*H*-indol-5-yl)-1-benzyl-5-fluoro-1*H*-indole-2-carboxamide;
*N*-(1-Methyl-1*H*-indol-5-yl)-1-{2-[3-(trifluoromethyl)-phenyl]ethyl}-5-fluoro-1*H*-indole-2-carboxamide;
*N*-(1-Methyl-1*H*-indol-5-yl)-1-[2-(3-fluorophenyl)ethyl]-5-fluoro-1*H*-indole-2-carboxamide.

**8.** Process for the preparation of a compound of formula (I) according to Claim 1, **characterized in that** a compound of general formula (IV)

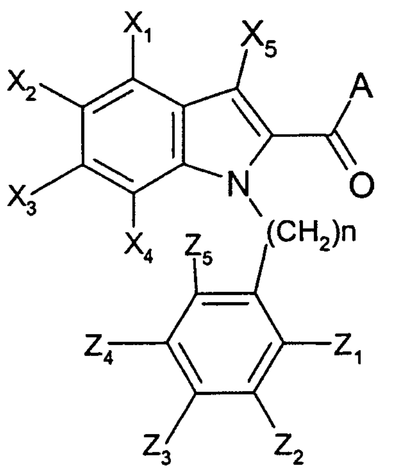

**(IV)**

in which $X_1$, $X_2$, $X_3$, $X_4$, $X_5$, $Z_1$, $Z_2$, $Z_3$, $Z_4$, $Z_5$ and n are as defined in the general formula (I) according to Claim 1 and A represents a $C_1$-$C_4$ alkoxy group,
is reacted with an amide of the compound of general formula (V)

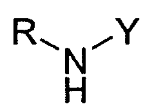

**(V)**

in which R and Y are as defined in the general formula (I) according to Claim 1,
at reflux of a solvent, the amide of the compound of general formula (V) being prepared by prior reaction of trimethylaluminium with the aminoindoles of general formula (V).

**9.** Process for the preparation of a compound of formula (I) according to Claim 1, **characterized in that** a compound of general formula (IV)

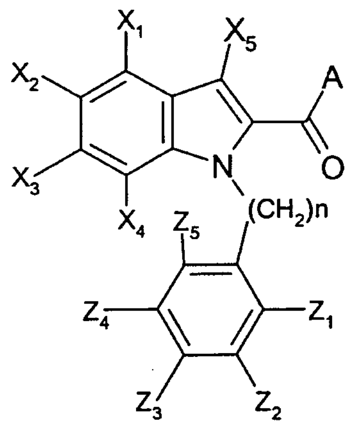

(IV)

in which $X_1$, $X_2$, $X_3$, $X_4$, $X_5$, $Z_1$, $Z_2$, $Z_3$, $Z_4$, $Z_5$ and n are as defined in the general formula (I) according to Claim 1 and A represents a hydroxyl group,
is converted to the acid chloride by the action of thionyl chloride at reflux of a solvent,
and then **in that** the compound of general formula (IV) obtained, in which $X_1$, $X_2$, $X_3$, $X_4$, $X_5$, $Z_1$, $Z_2$, $Z_3$, $Z_4$, $Z_5$ and n are as defined in the general formula (I) according to Claim 1 and A represents a chlorine atom, is reacted, in the presence of a base, with the aminoindole of general formula (V),

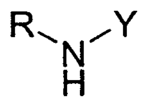

(V)

in which R and Y are as defined in the general formula (I) according to Claim 1,
or else **in that** a coupling reaction is carried out between a compound of general formula (IV), in which $X_1$, $X_2$, $X_3$, $X_4$, $X_5$, $Z_1$, $Z_2$, $Z_3$, $Z_4$, $Z_5$ and n are as defined in the general formula (I) according to Claim 1 and A represents a hydroxyl group, and the aminoindole of general formula (V), in which R and Y are as defined in the general formula (I) according to Claim 1, in the presence of a coupling agent and of a base in a solvent.

**10.** Medicament, **characterized in that** it comprises a compound of formula (I) according to any one of Claims 1 to 7 or a pharmaceutically acceptable salt or also a hydrate or a solvate of the compound of formula (I).

**11.** Pharmaceutical composition, **characterized in that** it comprises a compound of formula (I) according to any one of Claims 1 to 7, or a pharmaceutically acceptable salt, a hydrate or a solvate of this compound, and at least one pharmaceutically acceptable excipient.

**12.** Use of a compound of formula (I) according to any one of Claims 1 to 7 in the preparation of a medicament intended to prevent or to treat pathologies in which receptors of TRPV1 type are involved.

**13.** Use of a compound of formula (I) according to Claim 12 in the preparation of a medicament intended to prevent or to treat pain and inflammation, urological disorders, gynaecological disorders, gastrointestinal disorders, respiratory disorders, psoriasis, pruritus, irritation of the skin, eyes or mucous membranes, herpes or shingles or to treat depression.

**14.** Use of a compound of formula (I) according to Claim 13, **characterized in that** the pain and inflammation are chronic, neuropathic (traumatic, diabetic, metabolic, infectious, toxic, induced by an anticancer treatment or iatrogenic), (osteo)arthritic or rheumatic in nature or else of the following types: fibromyalgia, back pain, cancer-related pain, trigeminal neuralgia, cephalalgia, migraine, dental pain, burns, sunburn, bites or stings, post-herpetic neuralgia, muscle pain, nerve compression (central and/or peripheral), marrow and/or brain trauma, ischaemia (of the marrow

and/or brain), neurodegeneration, haemorrhagic vascular accidents (of the marrow and/or brain) or post-stroke pain.

**15.** Use of a compound of formula (I) according to Claim 13, **characterized in that** the urological disorders are chosen from bladder hyperactivity, bladder hyperreflexia, bladder instability, incontinence, urgent urination, urinary incontinence, cystitis, renal colic, pelvic hypersensitivity and pelvic pain.

**16.** Use of a compound of formula (I) according to Claim 13, **characterized in that** the gynaecological disorders are chosen from vulvodynia, salpingitis-related pain or dysmenorrhoea.

**17.** Use of a compound of formula (I) according to Claim 13, **characterized in that** the gastrointestinal disorders are chosen from gastro-oesophageal reflux disorder, stomach ulcers, duodenal ulcers, functional dyspepsia, colitis, IBS, Crohn's disease, pancreatitis, oesophagitis or biliary colic.

**18.** Use of a compound of formula (I) according to Claim 13, **characterized in that** respiratory disorders are chosen from asthma, coughs, COPD, bronchoconstriction and inflammatory disorders.

**Patentansprüche**

**1.** Verbindung der Formel (I),

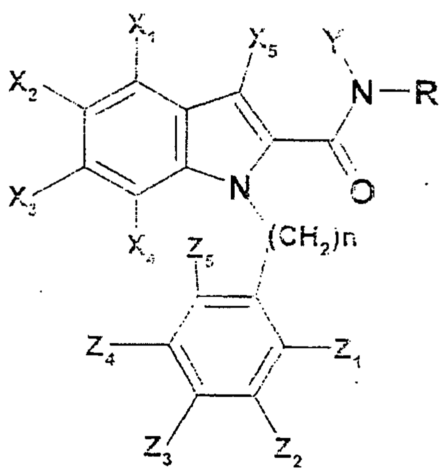

(I)

in der

$X_1$, $X_2$, $X_3$, $X_4$, $Z_1$, $Z_2$, $Z_3$, $Z_4$ und $Z_5$ unabhängig voneinander ein Wasserstoffatom oder ein Halogenatom oder eine $C_1$-$C_6$-Alkyl-, $C_3$-$C_7$-Cycloalkyl-, $C_1$-$C_6$-Fluoralkyl-, $C_1$-$C_6$-Alkoxy-, $C_1$-$C_6$-Fluoralkoxy-, Cyano-, $C(O)NR_1R_2$-, Nitro-, $NR_1R_2$-, $C_1$-$C_6$-Thioalkyl-, -S(O)-$C_1$-$C_6$-Alkyl-, -$S(O)_2$-$C_1$-$C_6$-Alkyl-, $SO_2NR_1R_2$-, $NR_3COR_4$-, $NR_3SO_2R_5$- oder Arylgruppe bedeuten;

$X_5$ ein Wasserstoffatom oder ein Halogenatom oder eine $C_1$-$C_6$-Alkyl-, $C_1$-$C_6$-Fluoralkylgruppe bedeutet;

R eine 4-, 5-, 6- oder 7-Indolylgruppe bedeutet,

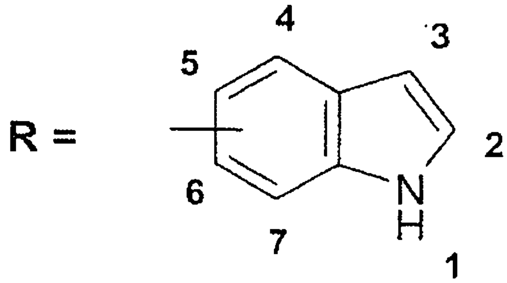

wobei R gegebenenfalls in 1-, 2- und/oder 3-Stellung durch eine oder mehrere Gruppen ausgewählt aus $C_1$-$C_6$-Alkylgruppen und $C_1$-$C_6$-Fluoralkylgruppen substituiert ist;
wobei R gegebenenfalls in 4-, 5-, 6- und/oder 7-Stellung durch eine oder mehrere Gruppen ausgewählt aus der Reihe Halogenatome, $C_1$-$C_6$-Alkyl-, $C_1$-$C_6$-Fluoralkyl-, $C_1$-$C_6$-Alkoxy-, $C_1$-$C_6$-Fluoralkoxygruppen substituiert ist; Y ein Wasserstoffatom oder eine $C_1$-$C_6$-Alkylgruppe bedeutet;
n gleich 0, 1, 2 oder 3 ist;
$R_1$ und $R_2$ unabhängig voneinander ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkyl- oder Arylgruppe bedeuten; oder $R_1$ und $R_2$ gemeinsam mit dem Stickstoffatom, an das sie gebunden sind, eine Acetidin-, Pyrrolidin-, Piperidin-, Azepin-, Morpholin-, Thiomorpholin-, Piperazin-, Homopiperazingruppe bilden, wobei diese Gruppe gegebenenfalls durch eine $C_1$-$C_6$-Alkyl-, $C_3$-$C_7$-Cycloalkyl-, $C_3$-$C_7$-Cycloalkyl-$C_1$-$C_3$-alkyl- oder Arylgruppe substituiert ist;
$R_3$ und $R_4$ unabhängig voneinander ein Wasserstoffatom oder eine $C_1$-$C_6$-Alkyl- oder Arylgruppe bedeuten;
$R_5$ eine $C_1$-$C_6$-Alkyl- oder Arylgruppe bedeutet;
als Base oder als Säureadditionssalz, sowie als Hydrat oder Solvat.

2. Verbindung der Formel (I) nach Anspruch 1, **dadurch gekennzeichnet daß**
$X_1$, $X_2$, $X_3$, $X_4$, $Z_1$, $Z_2$, $Z_3$, $Z_4$ und $Z_5$ unabhängig voneinander ein Wasserstoff- oder Halogenatom oder eine $C_1$-$C_6$-Alkyl-, $C_3$-$C_7$-Cycloalkyl-, $C_1$-$C_6$-Fluoralkyl-, $C_1$-$C_6$-Alkoxy-, $C_1$-$C_6$-Fluoralkoxy-, Nitro-, $NR_1R_2$-, $C_1$-$C_6$-Thioalkyl-, -S(O)-$C_1$-$C_6$-Alkyl-, -$S(O)_2$-$C_1$-$C_6$-Alkyl- oder Arylgruppe bedeuten;
$X_5$ ein Wasserstoffatom oder eine $C_1$-$C_6$-Alkylgruppe bedeutet;
R eine 4-, 5-, 6- oder 7-Indolylgruppe bedeutet,

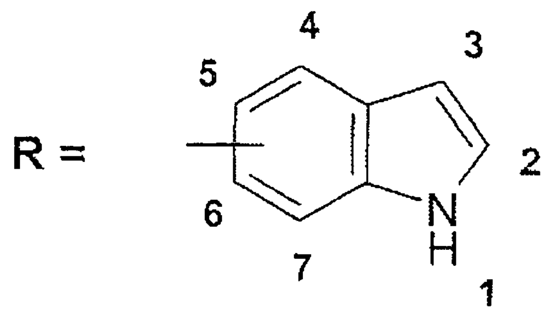

wobei R gegebenenfalls in 1-, 2- und/oder 3-Stellung durch eine oder mehrere $C_1$-$C_6$-Alkylgruppen substituiert ist;
Y ein Wasserstoffatom bedeutet;
n gleich 0, 1, 2 oder 3 ist;
$R_1$ und $R_2$ unabhängig voneinander ein Wasserstoffatom bedeuten;
als Base oder als Säureadditionssalz, sowie als Hydrat oder Solvat.

3. Verbindung der Formel (I) nach Anspruch 1 oder 2, **dadurch gekennzeichnet, daß** R eine Indol-5-ylgruppe bedeutet,

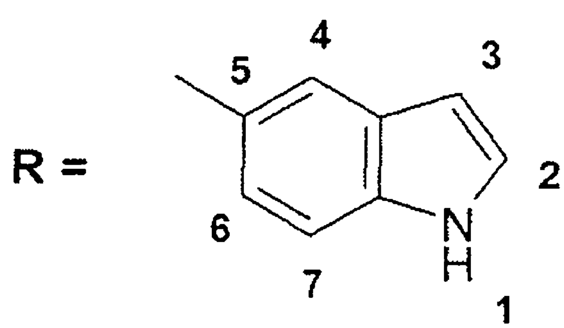

wobei R gegebenenfalls in 1-, 2- und/oder 3-Stellung durch eine oder mehrere Gruppen ausgewählt aus $C_1$-$C_6$-Alkylgruppen und $C_1$-$C_6$-Fluoralkylgruppen substituiert ist;
wobei R gegebenenfalls in 4-, 6-, und/oder 7-Stellung durch eine oder mehrere Gruppen ausgewählt aus der Reihe

Halogenatome, $C_1$-$C_6$-Alkyl-, $C_1$-$C_6$-Fluoralkyl-, $C_1$-$C_6$-Alkoxy-, $C_1$-$C_6$-Fluoralkoxygruppen substituiert ist; als Base oder als Säureadditionssalz, sowie als Hydrat oder Solvat.

**4.** Verbindung der Formel (I) nach einem der Ansprüche 1 bis 3, **dadurch gekennzeichnet, daß** $X_2$ und/oder $X_3$ nicht ein Wasserstoffatom bedeuten;
als Base oder als Säureadditionssalz, sowie als Hydrat oder Solvat.

**5.** Verbindung der Formel (I) nach einem der Ansprüche 1 bis 4, **dadurch gekennzeichnet, daß** $X_5$ ein Wasserstoffatom bedeutet;
als Base oder als Säureadditionssalz, sowie als Hydrat oder Solvat.

**6.** Verbindung der Formel (I) nach einem der Ansprüche 1 bis 5, **dadurch gekennzeichnet, daß** Y ein Wasserstoffatom bedeutet;
als Base oder als Säureadditionssalz, sowie als Hydrat oder Solvat.

**7.** Verbindung der Formel (I) nach Anspruch 1, ausgewählt aus:

*N*-(1-Methyl-1*H*-indol-5-yl)-1-(3-trifluormethylbenzyl)-1*H*-indol-2-carboxamid;
*N*-(1-Methyl-1*H*-indol-5-yl)-5-methoxy-1-(3-trifluormethylbenzyl)-1*H*-ndol-2-carboxamid;
*N*-(1-Methyl-1*H*-indol-5-yl)-5-fluor-1-(3-fluorbenzyl)-1*H*-indol-2-carboxamid;
*N*-(1-Methyl-1*H*-indol-5-yl)-1-(3-trifluormethylphenyl)-1*H*-indol-2-carboxamid;
*N*-(1-Methyl-1*H*-indol-5-yl)-1-(3,5-dimethylphenyl)-1*H*-indol-2-carboxamid;
*N*-(1-Methyl-1*H*-indol-5-yl)-5-methyl-1-(3-trifluormethylbenzyl)-1*H*-indol-2-carboxamid;
*N*-(1-Methyl-1*H*-indol-5-yl)-1-phenyl-1*H*-indol-2-carboxamid;
*N*-(1-Methyl-1*H*-indol-5-yl)-6-methoxy-1-(3-trifluormethylbenzyl)-1*H*-indol-2-carboxamid;
*N*-(1-Methyl-1*H*-indol-5-yl)-5-chlor-1-(3-trifluormethylbenzyl)-1*H*-indol-2-carboxamid;
*N*-(1-Methyl-1*H*-indol-5-yl)-4-methoxy-1-(3-trifluormethylbenzyl)-1*H*-indol-2-carboxamid;
*N*-(1-Methyl-1*H*-indol-5-yl)-5-fluor-1-(3-trifluormethylbenzyl)-1*H*-indol-2-carboxamid;
*N*-(1-Methyl-1*H*-indol-5-yl)-5-fluor-1-(4-trifluormethylbenzyl)-1*H*-indol-2-carboxamid;
*N*-(1-Methyl-1*H*-indol-5-yl)-1-(6-chlor-3-trifluormethylbenzyl)-5-fluor-1*H*-indol-2-carboxamid;
*N*-(1-Methyl-1*H*-indol-5-yl)-5-fluor-1-(3-methylbenzyl)-1*H*-indol-2-carboxamid;
*N*-(1-Methyl-1*H*-indol-5-yl)-5-fluor-1-(3-methoxybenzyl)-1*H*-indol-2-carboxamid;
*N*-(1-Methyl-1*H*-indol-5-yl)-5-fluor-1-(3-trifluormethoxybenzyl)-1*H*-indol-2-carboxamid;
*N*-(1-Methyl-1*H*-indol-5-yl)-1-(4-*tert*-butylbenzyl)-5-fluor-1*H*-indol-2-carboxamid;
*N*-(1-Methyl-1*H*-indol-5-yl)-1-(3-chlorbenzyl)-5-fluor-1*H*-indol-2-carboxamid;
*N*-(1-Methyl-1*H*-indol-5-yl)-1-(4-fluorphenyl)-1*H*-indol-2-carboxamid;
*N*-(1-Methyl-1*H*-indol-5-yl)-1-(4-methylthiophenyl)-1*H*-indol-2-carboxamid;
*N*-(1-Methyl-1*H*-indol-5-yl)-1-(4-*sec*-butylphenyl)-1*H*-indol-2-carboxamid;
*N*-(1-Methyl-1*H*-indol-5-yl)-5-methoxy-1-phenyl-1*H*-indol-2-carboxamid;
*N*-(1-Methyl-1*H*-indol-5-yl)-1-(4-isopropylphenyl)-6-methyl-1*H*-indol-2-carboxamid;
*N*-(1-Methyl-1*H*-indol-5-yl)-1-(4-n-pentylphenyl)-1*H*-indol-2-carboxamid;
*N*-(1-Methyl-1*H*-indol-5-yl)-1-(4-cyclopentylphenyl)-1*H*-indol-2-carboxamid;
*N*-(1-Methyl-1*H*-indol-5-yl)-1-(biphenyl-4-yl)-1*H*-indol-2-carboxamid;
*N*-(1-Methyl-1*H*-indol-5-yl)-1-(4-trifluormethylphenyl)-1*H*-indol-2-carboxamid;
*N*-(1-Methyl-1*H*-indol-5-yl)-1-(4-*n*-propylphenyl)-1*H*-indol-2-carboxamid;
*N*-(1-Methyl-1*H*-indol-5-yl)-1-(3-methylphenyl)-1*H*-indol-2-carboxamid;
*N*-(1-Methyl-1*H*-indol-5-yl)-1-(4-isopropylphenyl)-1*H*-indol-2-carboxamid;
*N*-(1-Methyl-1*H*-indol-5-yl)-1-(4-*tert*-butylphenyl)-1*H*-indol-2-carboxamid;
*N*-(1-Methyl-1*H*-indol-5-yl)-1-(4-cyclohexylphenyl)-1*H*-indol-2-carboxamid;
*N*-(1-Methyl-1*H*-indol-5-yl)-1-(4-ethoxyphenyl)-1*H*-indol-2-carboxamid;
*N*-(1-Methyl-1*H*-indol-5-yl)-1-(4-chlorphenyl)-1*H*-indol-2-carboxamid;
*N*-(1-Methyl-1*H*-indol-5-yl)-1-(3,5-difluorbenzyl)-5-fluor-1H-indol-2-carboxamid;
*N*-(1-Methyl-1*H*-indol-5-yl)-1-(2-fluorbenzyl)-5-fluor-1*H*-indol-2-carboxamid;
*N*-(1-Methyl-1*H*-indol-5-yl)-1-(4-trifluormethoxybenzyl)-5-fluor-1*H*-indol-2-carboxamid;
*N*-(1-Methyl-1*H*-indol-5-yl)-1-(4-brombenzyl)-5-fluor-1*H*-indol-2-carboxamid;
*N*-(1-Methyl-1*H*-indol-5-yl)-1-(3,4-dimethylphenyl)-1*H*-indol-2-carboxamid;
*N*-(1-Methyl-1*H*-indol-5-yl)-1-(phenylethyl)-5-fluor-1*H*-indol-2-carboxamid;
*N*-(1-Methyl-1*H*-indol-5-yl)-1-(3-isopropylphenyl)-5-trifluormethoxy-1*H*-indol-2-carboxamid;

*N*-(1-Methyl-1*H*-indol-5-yl)-1-(3-trifluormethylbenzyl)-5-trifluormethyl-1*H*-indol-2-carboxamid;
*N*-(1-Methyl-1*H*-indol-5-yl)-1-(3-trifluormethylphenyl)-5-fluor-1H-indol-2-carboxamid;
*N*-(1-Methyl-1*H*-indol-5-yl)-1-(3-fluorbenzyl)-5-trifluormethyl-1H-indol-2-carboxamid;
*N*-(1-Methyl-1*H*-indol-6-yl)-1-(3,5-dimethylphenyl)-1*H*-indol-2-carboxamid;
*N*-(1,2,3-Trimethyl-1*H*-indol-5-yl)-1-(3,5-dimethylphenyl)-1*H*-indol-2-carboxamid;
*N*-(1-Methyl-1*H*-indol-4-yl)-1-(3,5-dimethylphenyl)-1*H*-indol-2-carboxamid;
*N*-(1-Methyl-1*H*-indol-6-yl)-1-(3-fluorbenzyl)-5-fluor-1*H*-indol-2-carboxamid;
*N*-(1-Methyl-1*H*-indol-7-yl)-1-(3-fluorbenzyl)-5-fluor-1*H*-indol-2-carboxamid;
*N*-(1-Methyl-1*H*-indol-5-yl)-1-[2-(2-fluorphenyl)ethyl]-5-fluor-1*H*-indol-2-carboxamid;
*N*-(1-Methyl-1*H*-indol-5-yl)-1-[2-(4-fluorphenyl)ethyl]-5-fluor-1*H*-indol-2-carboxamid;
*N*-(1-Methyl-1*H*-indol-5-yl)-1-(3-phenylpropyl)-5-fluor-1*H*-indol-2-carboxamid;
*N*-(1-Methyl-1*H*-indol-5-yl)-1-(3-fluorbenzyl)-5-methoxy-1*H*-indol-2-carboxamid;
*N*-(1-Methyl-1*H*-indol-7-yl)-1-(3,5-dimethylphenyl)-1*H*-indol-2-carboxamid;
*N*-(1,2,3-Trimethyl-1*H*-indol-5-yl)-1-(3-fluorbenzyl)-5-fluor-1*H*-indol-2-carboxamid;
*N*-(1-Methyl-1*H*-indol-4-yl)-1-(3-fluorbenzyl)-5-fluor-1*H*-indol-2-carboxamid;
*N*-(1,2-Dimethyl-1*H*-indol-5-yl)-1-(3-fluorbenzyl)-5-fluor-1*H*-indol-2-carboxamid;
*N*-(1-Methyl-1*H*-indol-5-yl)-1-[2-(4-tert-butylphenyl)ethyl]-5-fluor-1*H*-indol-2-carboxamid;
*N*-(1-Methyl-1*H*-indol-5-yl)-1-(3-fluorbenzyl)-6-methoxy-1*H*-indol-2-carboxamid;
*N*-(1-Methyl-1*H*-indol-5-yl)-1-(3-fluorbenzyl)-4-methoxy-1*H*-indol-2-carboxamid;
*N*-(1-Methyl-1*H*-indol-5-yl)-1-(3-fluorbenzyl)-4,6-dimethoxy-1*H*-indol-2-carboxamid;
*N*-(1-Methyl-1*H*-indol-5-yl)-1-(3-fluorbenzyl)-5-chlor-1*H*-indol-2-carboxamid;
*N*-(1-Methyl-1*H*-indol-5-yl)-1-(3-fluorbenzyl)-5-methyl-1*H*-indol-2-carboxamid;
*N*-(1-Methyl-1*H*-indol-5-yl)-1-(3-fluorbenzyl)-5-methylsulfonyl-1*H*-indol-2-carboxamid;
*N*-(1-Methyl-1*H*-indol-5-yl)-1-(3-fluorbenzyl)-5-nitro-1*H*-indol-2-carboxamid;
*N*-(1-Isopropyl-1*H*-indol-5-yl)-1-(3-fluorbenzyl)-5-fluor-1*H*-indol-2-carboxamid;
*N*-(1-Methyl-1*H*-indol-5-yl)-1-(3-fluorbenzyl)-4-fluor-1*H*-indol-2-carboxamid;
*N*-(1-Methyl-1*H*-indol-5-yl)-1-(3-fluorbenzyl)-5-isopropyl-1*H*-indol-2-carboxamid;
*N*-(1-Methyl-1*H*-indol-5-yl)-1-benzyl-5-trifluormethyl-1*H*-indol-2-carboxamid;
*N*-(1*H*-Indol-5-yl)-5-fluor-1-(3-fluorbenzyl)-1*H*-indol-2-carboxamid;
*N*-(1-Methyl-1*H*-indol-5-yl)-1-(3-fluorbenzyl)-5-trifluormethoxy-1*H*-indol-2-carboxamid;
*N*-(1-Methyl-1*H*-indol-5-yl)-1-(3-fluorbenzyl)-4-methyl-1*H*-indol-2-carboxamid;
*N*-(1-Methyl-1*H*-indol-5-yl)-1-(3-fluorbenzyl)-5-tert-butyl-1*H*-indol-2-carboxamid;
*N*-(1-Methyl-1*H*-indol-5-yl)-1-(3-fluorbenzyl)-5-amino-1*H*-indol-2-carboxamid;
*N*-(1-Methyl-1*H*-indol-5-yl)-1-(3-fluorbenzyl)-6-methyl-1*H*-indol-2-carboxamid;
*N*-(1-Methyl-1*H*-indol-5-yl)-1-(3-fluorbenzyl)-6-fluor-1*H*-indol-2-carboxamid;
*N*-(1-Methyl-1*H*-indol-5-yl)-1-(3-fluorbenzyl)-5,6-dimethoxy-1*H*-indol-2-carboxamid;
*N*-(1-Methyl-1*H*-indol-5-yl)-1-(3-fluorbenzyl)-1*H*-indol-2-carboxamid;
*N*-(1-Methyl-1*H*-indol-5-yl)-1-benzyl-5-fluor-1*H*-indol-2-carboxamid;
*N*-(1-Methyl-1*H*-indol-5-yl)-1-[2-(3-trifluormethylphenyl)ethyl]-5-fluor-1*H*-indol-2-carboxamid;
*N*-(1-Methyl-1*H*-indol-5-yl)-1-[2-(3-fluorphenyl)ethyl]-5-fluor-1H-indol-2-carboxamid.

**8.** Verfahren zur Herstellung einer Verbindung der Formel (I) nach Anspruch 1, **dadurch gekennzeichnet, daß** man eine Verbindung der allgemeinen Formel (IV)

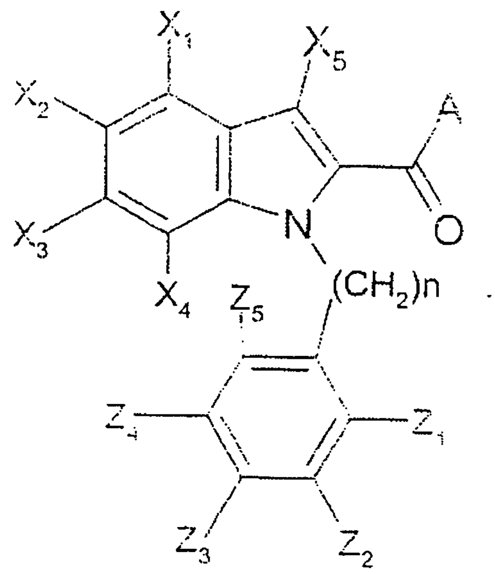

(IV)

in der $X_1$, $X_2$, $X_3$, $X_4$, $X_5$, $Z_1$, $Z_2$, $Z_3$, $Z_4$, $Z_5$ und n wie in der allgemeinen Formel (I) nach Anspruch 1 definiert sind und A eine $C_1$-$C_4$-Alkoxygruppe bedeutet,
mit einem Amid der Verbindung der allgemeinen Formel (V),

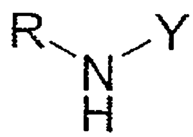

(V)

in der R und Y wie in der allgemeinen Formel (I) nach Anspruch 1 definiert sind,
unter Rückfluß eines Lösungsmittels umsetzt, wobei das Amid der Verbindung der allgemeinen Formel (V) durch vorheriges Einwirken von Trimethylaluminium auf die Aminoindole der allgemeinen Formel (V) hergestellt wird.

**9.** Verfahren zur Herstellung einer Verbindung der Formel (I) nach Anspruch 1, **dadurch gekennzeichnet, daß** man eine Verbindung der allgemeinen Formel (IV),

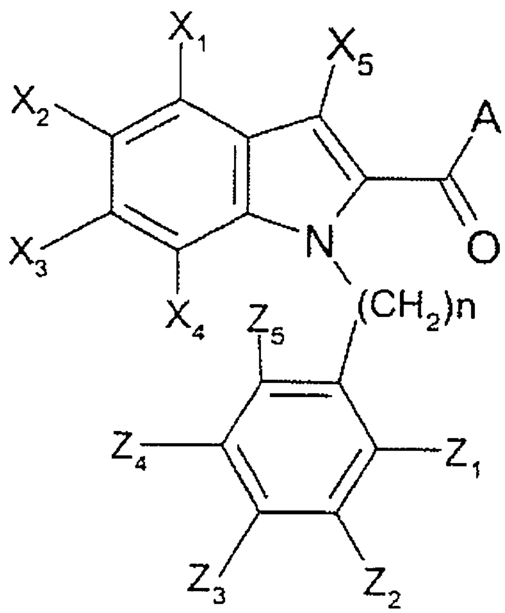

**(IV)**

in der $X_1$, $X_2$, $X_3$, $X_4$, $X_5$, $Z_1$, $Z_2$, $Z_3$, $Z_4$, $Z_5$ und n wie in der allgemeinen Formel (I) nach Anspruch 1 definiert sind und A eine Hydroxygruppe bedeutet,
durch Einwirken von Thionylchlorid unter Rückfluß eines Lösungsmittels in das Säurechlorid umwandelt,
und anschließend **dadurch**, daß man die erhaltene Verbindung der allgemeinen Formel (IV), in der $E_1$, $X_2$, $X_3$, $X_4$, $X_5$, $Z_1$, $Z_2$, $Z_3$, $Z_4$, $Z_5$ und n wie in der allgemeinen Formel (I) nach Anspruch 1 definiert sind und A ein Chloratom bedeutet, in Gegenwart einer Base mit einem Aminoindol der allgemeinen Formel (V),

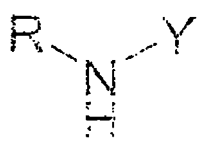

**(V)**

in der R und Y wie in der allgemeinen Formel (I) nach Anspruch 1 definiert sind, umsetzt,
oder **dadurch**, daß man eine Kopplung zwischen einer Verbindung der allgemeinen Formel (IV), in der $X_1$, $X_2$, $X_3$, $X_4$, $X_5$, $Z_1$, $Z_2$, $Z_3$, $Z_4$, $Z_5$ und n wie in der allgemeinen Formel (I) nach Anspruch 1 definiert sind und A eine Hydroxygruppe bedeutet, mit dem Aminoindol der allgemeinen Formel (V), in der R und Y wie in der allgemeinen Formel (I) nach Anspruch 1 definiert sind, in Gegenwart eines Kopplungsmittels und einer Base in einem Lösungsmittel durchführt.

**10.** Arzneimittel, **dadurch gekennzeichnet, daß** es eine Verbindung der Formel (I) nach einem der Ansprüche 1 bis 7 oder ein pharmazeutisch unbedenkliches Salz oder ein Hydrat oder ein Solvat der Verbindung der Formel (I) enthält.

**11.** Pharmazeutische Zusammensetzung, **dadurch gekennzeichnet, daß** sie eine Verbindung der Formel (I) nach einem der Ansprüche 1 bis 7 oder ein pharmazeutisch annehmbares Salz oder ein Hydrat oder ein Solvat dieser Verbindung sowie mindestens einen pharmazeutisch unbedenklichen Träger enthält.

**12.** Verwendung einer Verbindung der Formel (I) nach einem der Ansprüche 1 bis 7 für die Herstellung eines Arzneimittels zur Vorbeugung oder Behandlung von Pathologien, an denen die Rezeptoren des TRPV1-Typs beteiligt sind.

**13.** Verwendung einer Verbindung der Formel (I) nach Anspruch 12 für die Herstellung eines Arzneimittels zur Vorbeugung oder Behandlung von Schmerz und Entzündung, Störungen der Harnwege, Störungen gynäkologischer Art, Störungen des Magen-Darm-Trakts, Störungen der Atemwege, Schuppenflechte, Juckreiz, Reizungen der Haut, der Augen oder der Schleimhäute, Herpes, Gürtelrose oder zur Behandlung von Depression.

**14.** Verwendung einer Verbindung der Formel (I) nach Anspruch 13, **dadurch gekennzeichnet, daß** der Schmerz und die Entzündung chronisch, neuropathisch (traumatisch, diabetisch, metabolisch, infektiös, toxisch, durch Behandlung von Krebs induziert oder iatrogen), (osteo)arthritisch, rheumatisch oder vom Fibromyalgie-Typus, Rückenschmerzen, mit Krebs in Zusammenhang stehendem Schmerz, Gesichtsneuralgie, Kopfschmerzen, Migräne, Zahnschmerz, Verbrennung, Sonnenstich, Biß- oder Stichwunden, Post-Herpes-Neuralgie, Muskelschmerz, Nervenkompression (zentral und/oder peripher), Traumen des Rückenmarks und/oder Hirns, Ischämie (des Rückenmarks und/oder des Hirns), Neurodegeneration, hämorrhagische Gefäßverletzungen (des Rückenmarks und/oder Hirns), Schmerzen nach Herzschlag sind.

**15.** Verwendung einer Verbindung der Formel (I) nach Anspruch 13, **dadurch gekennzeichnet, daß** die Störungen der Harnwege aus der folgenden Reihe ausgewählt sind: Hyperaktivität der Blase, Blasen-Hyperreflexie, Blaseninstabilität, Inkontinenz, Harndrang, Harninkontinenz, Cystitis, Nierenkolik, Hypersensitivität des Beckens und Bekkenschmerzen.

**16.** Verwendung einer Verbindung der Formel (I) nach Anspruch 13, **dadurch gekennzeichnet, daß** die Störungen gynäkologischer Art aus der folgenden Reihe stammen: Vulvodynie, mit Eileiterentzündung oder mit Dysmenorrhoe in Zusammenhang stehende Schmerzen.

**17.** Verwendung einer Verbindung der Formel (I) nach Anspruch 13, **dadurch gekennzeichnet, daß** die Störungen des Magen-Darm-Trakts aus der folgenden Reihe stammen: Störung des gastroösophagealen Reflux, Magengeschwür, Zwölffingerdarmgeschwür, funktionelle Dyspesie, Colitis, IBS, Morbus Crohn, Pankreatitis, Ösophagitis, Leberkolik.

**18.** Verwendung einer Verbindung der Formel (I) nach Anspruch 13, **dadurch gekennzeichnet, daß** die Störungen der Atemwege aus der folgenden Reihe stammen: Asthma, Husten, COPD, Bronchokonstriktion und entzündliche Störungen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03049702 A
- WO 9205170 A


### Littérature non-brevet citée dans la description


- **KOLASA T.** *Bioorg.Med.Chem.,* 1997, vol. 5 (3), 507
- **ABRAMOVITCH R.** *Synth. Commun.,* 1995, vol. 25 (1), 1
- **MURAKAMI Y.** *Chem.Pharm.Bull.,* 1995, vol. 43 (8), 1281
- **S.L. BUCHWALD.** *J.Am.Chem.Soc,* 2002, vol. 124, 11684
- **W.W.K.R. MEDERSKI.** *Tetrahedron,* 1999, vol. 55, 12757
- **D. KNITTEL.** *Synthesis,* 1985, vol. 2, 186
- **T.M. WILLIAMS.** *J.Med.Chem.,* 1993, vol. 36 (9), 1291
- **I.T. FORBES.** *J.Med.Chem.,* 1993, vol. 36 (8), 1104
- *Pharmazie,* 1990, vol. 45, 346
- **I.T. FORBES.** *J.Med.Chem,* 1993, vol. 36 (8), 1104